# EUROPEAN PATENT APPLICATION

(11) **EP 2 508 186 A1**
(43) Date of publication of application: **10.10.2012**
(21) Application number: 11161325.3
(22) Date of filing: 06.04.2011
(51) Int. Cl.: A61K 31/538, C07D 265/38, A61P 25/28

(54) **Phenoxazinones or phenothiazones as inhibitors of amyloid formation**

(71) Applicant: Max-Delbrück-Centrum für Molekulare Medizin, 13125 Berlin (DE)
(72) Inventor: Wanker, Erich, 13187 Berlin (DE); Herbst, Martin, 10119 Berlin (DE); Bieschke, Jan, 10435 Berlin (DE); Wagner, Anne, 10439 Berlin (DE); Wiglenda, Thomas, 10367 Berlin (DE)
(74) Representative: Weiss, Wolfgang

(57) **Abstract**

The present invention relates to compounds useful in the treatment of amyloid diseases, such as Alzheimer's disease. Further, the compounds are useful for imaging amyloid deposits.

## Description

The present invention relates to compounds useful in the treatment of amyloid diseases, such as Alzheimer's disease. Further, the compounds are useful for imaging amyloid deposits.

Accumulation of extracellular amyloid plaques is a key feature of Alzheimer's disease (AD) pathogenesis (Soto, 1999). Amyloid plaques are mainly constituted of fibrils formed of amyloid-β (Aβ), an amphipathic polypeptide consisting of 39-42 amino acids. Aggregation-prone Aβ peptides are produced by proteolytic cleavage of the amyloid-β precursor protein (APP), a membrane protein of largely unknown cellular function (Walsh, 2007).

Soluble Aβ monomers self-assemble into insoluble amyloid fibrils by nucleation-dependent polymerization, a process that is accelerated by the addition of preformed amyloid fibrils (Harper, 1997). Amyloid formation typically involves the generation of different types of aggregate species whose structures, sizes and biological activities are not well defined (Dobson, 2003). In the initial phase, soluble spherical Aβ oligomers are formed by the self-assembly of monomers. These structures have a diameter of ∼15-25 nm (Bitan, 2003) and over time convert into larger aggregate species with a typical worm-like morphology and a length of ∼150-200 nm (Harper, 1997; Walsh, 1997; Harper, 1999), which are termed "protofibrils". They function as templates for the rapid assembly of mature amyloid aggregates with fibrillar morphology and a characteristic cross-β-sheet structure (Dobson, 2003).

Growing experimental evidence suggests that soluble, pre-fibrillar assemblies of Aβ cause memory impairment and are more toxic than end-stage, insoluble Aβ fibrils (Walsh, 2007; Bucciantini, 2002). However, due to the dynamic nature of protein aggregation and the difficulty in obtaining purified fractions of aggregation products, a clear relationship between the size and structure of amyloid species and their cellular toxicity has not been established.

Biochemical and cell biological studies indicate that amyloid formation pathways can be manipulated with small molecules (Herbst, 2006; Cohen, 2003). Organic osmolytes such as trimethylamine N-oxide or glycerol were shown to accelerate Aβ fibrillogenesis *in vitro* (Yang, 1999). In contrast, compounds such as dopamine or calmidazolium chloride were found to efficiently block amyloid fibrillogenesis (Williams, 2005; Conway, 2001; Walsh, 2002). Substances selectively inhibiting Aβ oligomerization (Necula, 2007), redirecting amyloid formation pathways (Ehrnhoefer, 2006; Ehrnhoefer, 2008) or remodeling preformed amyloid structures (Bieschke, 2010) were also reported. Thus, small molecules are powerful tools that allow the modulation of different events in amyloid formation cascades and can exert strong influence like stabilization or morphological/ structural conversion of well-defined aggregate species (LeVine, 2002).

If small diffusible aggregation products are indeed the toxic species in AD (Walsh, 2007), accelerating nucleation and fibril assembly with small molecules should reduce their toxicity in biological model systems. Thus, the present inventors have searched for compounds that promote Aβ polymerization using a membrane filter retardation assay.

Here, it is shown that the natural compound mixture orcein and the related substance 04 are potent stimulators of Aβ fibrillogenesis. Strikingly, compound-mediated amyloid polymerization was accompanied by a dramatic reduction of toxic on-pathway aggregation intermediates. Detailed biochemical and biophysical studies revealed that 04 directly binds to hydrophobic regions in the amyloid-β 1-42 (Aβ₄₂) peptide, which were shown previously to be critical for spontaneous amyloid polymerization (Kim and Hecht, 2006). Our data indicate that 04 binding to Aβ₄₂ peptides stabilizes the assembly of seeding-competent, β-sheet-rich protofilaments that efficiently nucleate the assembly of mature amyloid aggregates with fibrillar morphology (Harper, 1997).

A subject-matter of the present invention is a compound of formula (I), 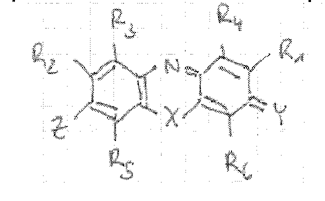 wherein X is O or S, Y is O or NR⁷, Z is OR⁷ or N(R⁷)₂,
wherein R⁷ is in each case independently selected from H and C₁₋₃ alkyl optionally substituted with halogen,
at least one of R¹ and R² is a C₃₋₈ cyclic group substituted with at least one OH group and optionally other substituents,
R¹, R², R³, R⁴, R⁵ and R⁶ are in each case independently selected from H, halogen or C₁₋₃ alkyl or C₁₋₃ alkoxy optionally substituted with halogen,
or a pharmaceutically acceptable salt thereof,
particularly for use in medicine.

The compounds of formula (I) are phenoxazines when X is O, or phenothiazines when X is S. Preferably, X is O.

The present invention encompasses compounds, wherein (i) Y is O and Z is OR⁷, (ii) Y is O and Z is N(R⁷)₂, (iii) Y is NR⁷and Z is OH or (iv) Y is NR⁷ and Z is N(R⁷)₂. R⁷may be in each case independently H or C₁₋₃ alkyl such as methyl, ethyl, n-propyl or i-propyl or mono- or polyhalogenated C₁₋₃ alkyl such as trifluoromethyl. Preferably R⁷ is H.

Preferably, Y is O and Z is OH. More preferably, X is O, Y is O and Z is OH.

R¹, R², R³, R⁴, R⁵ and R⁶ are in each case independently selected from H, halogen such as Cl, Br or I, or C₁₋₃ alkyl such as methyl, ethyl, n-propyl or i-propyl, optionally substituted with halogen, such as trifluoromethyl or C₁₋₃ alkoxy such as methoxy, ethoxy, propoxy, optionally mono- or polysubstituted with halogen such as trifluoromethoxy.

Preferably, R³, R⁴, R⁵ and R⁶ are in each case independently selected from H and methyl. More preferably R³, R⁴, R⁵ and R⁶ are H.

At least one of R¹ and R² is a C₃₋₈ cyclic group which is substituted with at least one, e.g. one, two or three OH groups and optionally other substituents such as halogen or C₁₋₃ alkyl or C₁₋₃ alkoxy, optionally substituted with halogen.

The cyclic group may be a saturated, unsaturated or aromatic group, which may comprise up to three heteroatoms selected from N and O. Preferably, the cyclic group is a C₅₋₆ aromatic or heteroaromatic group. More preferably, the cyclic group is a phenyl group, which may be substituted with one or two OH groups and optionally other substituents. Most preferably, R¹ and/or R² is a phenyl group substituted with two OH groups such as a 2,4-dihydroxyphenyl group.

In the compounds of formula (I), only R¹ may be a cyclic group, only R² may be a cyclic group or R¹ and R² may be both cyclic groups. Preferably, both R¹ and R² are cyclic groups, e.g. substituted phenyl groups as discussed above. Specific examples of the compounds of the present invention are selected from α-amino orcein, α-hydroxy orcein, β-amino orcein, β-amino orceimine, γ-amino orcein, γ-amino orceimine, 2,8-bis (2,4-dihydroxy-phenyl)-7-hydroxy-phenazin-3-one (04) or pharmaceutically acceptable salts thereof. An especially preferred compound is 04 which is commercially available from TimTec, Newark, USA or which can be synthesized as described in Example 1.

The compounds of the present invention are suitable for therapeutic or diagnostic applications. Particularly for diagnostic applications, the compound of formula (I) may comprise at least one detectable group, e.g. a radioactive or non-radioactive detectable group. In one embodiment, the detectable group is a deuterium atom, i.e. a compound wherein at least one hydrogen atom has been replaced by deuterium. Examples of radioactive substituents are or comprise ³H, isotopes of C, e.g. ¹⁴C, isotopes of I, e.g. ¹²³I, ¹²⁵I or ¹³¹I, isotopes of Br, e.g. ⁷⁵Br, ⁷⁶Br or ⁷⁷Br isotopes of F, e.g. ¹⁸F or ¹⁹F. In specific embodiments, the compound comprises at least one substituent which is an ¹⁸F or ¹⁹F atom or a group comprising an ¹⁸F or ¹⁹F atom, such as -CH₂-¹⁸F, -CH- ¹⁹F, -CH(¹⁸F)₂, -CH(¹⁹F)₂, -C¹⁸F₃, -C(¹⁹F)₃, -CH₂-CH₂-¹⁸F, (¹⁹F), O-CH₂-¹⁸F, (¹⁹F) O-CH₂-CH₂-¹⁸F, (¹⁹F), CH₂-CH₂-CH₂-¹⁸F, (¹⁹F), O-CH₂-CH₂-CH₂-¹⁸F, (¹⁹F) or aromatic compounds comprising at least one, e.g. 1-4 ¹⁸F or ¹⁹F groups. Such groups may be detected by suitable methods including MRS (Magnetic Resonance Spectroscopy), MRI (Magnetic Resonance Imaging), PET (Positron Emission Tomography), or SPECT (Single-Photon Emission Computed Tomography). Further, ESR (electron spin resonance) or EPR (electron paramagnetic resonance) methods may be employed, wherein a labelled compound is used in combination with the spin probes. Thereby, stable radicals in solutions are used (unpaired electrons), e.g. nitroxide radicals (nitroso-radicals). The probes used are for example TEMPO, TEMPOL (4-OH-TEMPO), TANONE, DTBN, NIT-R with R: isopropyl, ethyl, methyl, phenyl or DOXYL and PROXYL and derivatives thereof.

The compounds of formula (I) belong to the class of phenoxazines or phenothiazines. These compounds may be manufactured as described in the Examples.

The compounds of the present invention may accelerate amyloid-β fibril formation and/or inhibit amyloid-β oligomer formation. They can be used in medical applications as therapeutic agents, as diagnostic agents or in non-medical applications.

For medical applications, the compounds may be administered as such or as pharmaceutically acceptable salts. The term "salt" refers to pharmaceutically acceptable inorganic and organic acid addition salts of the compounds of formula (I). Representative salts include halogenide salts, such as bromide, chloride, sulfate, hydrogen-sulfate, nitrate, phosphate, carboxylate salts such as acetate, lactate, maleate, succinate, or sulfonate salts such as tosylate or mesylate. Further salts may include cations based on alkali- and alkaline earth metals such as sodium, lithium, potassium, calcium, magnesium as well as organic cations such as ammonium, quaternary ammonium or amine cations.

Thus, the compounds are suitable for medical applications, e.g. in human or veterinary medicine. Particularly the compounds may be used in the treatment, diagnosis and/or monitoring of a disease associated with, accompanied by and/or caused by protein misfolding, particularly amyloid-β misfolding or formation of protein aggregates, particularly of amyloid-β aggregates.

The disease may e.g. be selected from Alzheimer's disease, Parkinson's disease, an amyloidosis such as αβ-amyloidosis, primary systemic amyloidosis, secondary systemic amyloidosis, senile systemic amyloidosis, familial amyloid polyneuropathy 1, hereditary cerebral amyloid angiopathy, hemodialysis-related amyloidosis, familial amyloid polyneuropathy III, Finnish hereditary systemic amyloidosis, type 2 diabetes, medullary carcinoma of thyroid, atrial amyloidosis, hereditary non-neuropathic systemic amyloidosis, injection-localized amyloidosis, hereditary renal amyloidosis, amyotrophic lateral sclerosis, schizophrenia, sickle cell anaemia, unstable haemoglobin inclusion body haemolysis, α1- antitrypsin deficiency, antithrombin deficiency, thromboembolic disease, spinobulbar muscular atrophy (Kennedy disease), dent-atorubral-pallidoluysian atrophy (Haw River syndrome), Huntington's disease, spinocerebellar ataxia type 1, spinocerebellar ataxia type 2, spinocerebellar ataxia type 3 (Machado-Joseph disease), spinocerebellar ataxia type 6, spinocerebellar ataxia type 7, spinocerebellar ataxia type 17, transmissible spongiform encephalopathies (prion diseases): Bovine spongiform encephalopathy, kuru, Gerstmann-Straeussler-Scheinker syndrome, fatal familial insomnia, scrapie, Creutzfeldt-Jakob disease and variant Creutzfeldt-Jakob disease. Particularly, the disease is an amyloidosis, more particularly β-amyloidosis.

The invention, however, also refers to a non-medical use of a compound of formula (I) as an accelerator of amyloid-β fibril formation and/or an inhibitor of amyloid-β oligomer formation surface properties, and/or as an activator of degradation of a misfolded protein or a protein aggregate. The protein is particularly selected from amyloid-proteins. More particularly, the protein is amyloid-β.

A further aspect of the present invention refers to a pharmaceutical and/or diagnostic composition comprising at least one compound of formula (I) and optionally a physiologically acceptable carrier, e.g. a carrier suitable for therapeutic or diagnostic applications. The composition may be in form of a solution, suspension, emulsion or solid substance, e.g. a tablet or capsule. Preferably, the composition is a dosage form suitable for oral administration.

Optionally, the composition may contain other active agents, e.g. therapeutic agents for the treatment of amyloid-associated diseases as indicated above. The composition of the present invention comprises at least one compound of formula (I) in a pharmaceutically or diagnostically effective amount. This amount may vary broadly according to the desired application from e.g. 0.01 mg to 1000 mg or even higher. The composition may be administered to a subject in need thereof, e.g. a human patient, by any suitable route, e.g. orally, topically, transdermally, rectally or parenterally, e.g. by intravenous, intraarterial, intramuscular, subcutaneous, intrathecal or intraperitoneal injection or infusion. Preferably, the composition is administered orally.

For diagnostic applications, it may be preferred to use a compound comprising at least one detectable group and to detect binding of the compound to amyloid species in vivo, e.g. as described above by MRI, PET or SPECT.

Further, the invention shall be explained in more detail by the following Figures and Examples.

### Figures

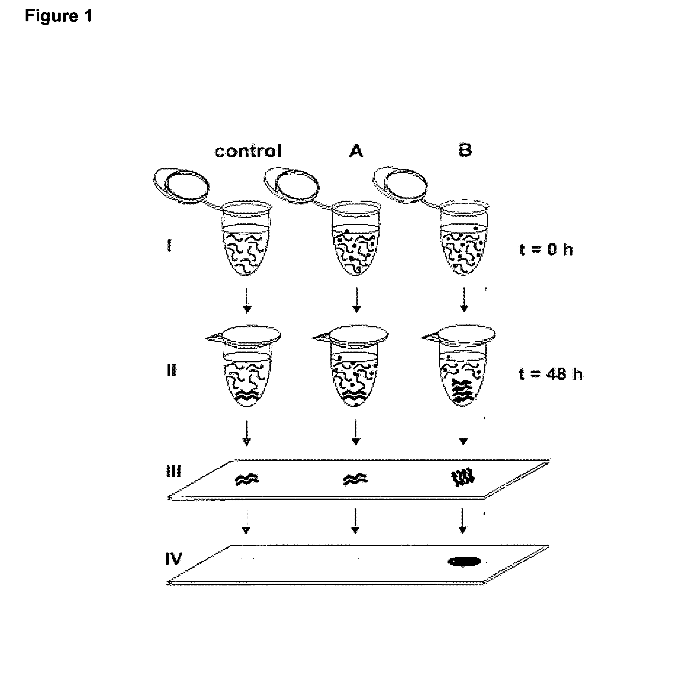

**Fig. 1****:** Detection of insoluble Aβ₄₂ aggregates by filter retardation assay. Peptides were incubated in the presence of test compounds (A and B) or solvent (control) (I and II). After different time points (e.g. 48 h) samples were denatured in SDS and passed through a cellulose acetate filter (III). Large aggregates, which are resistant against SDS treatment, were retained on the filter membrane and quantified using the Aβ-specific antibody 6E10 (IV).

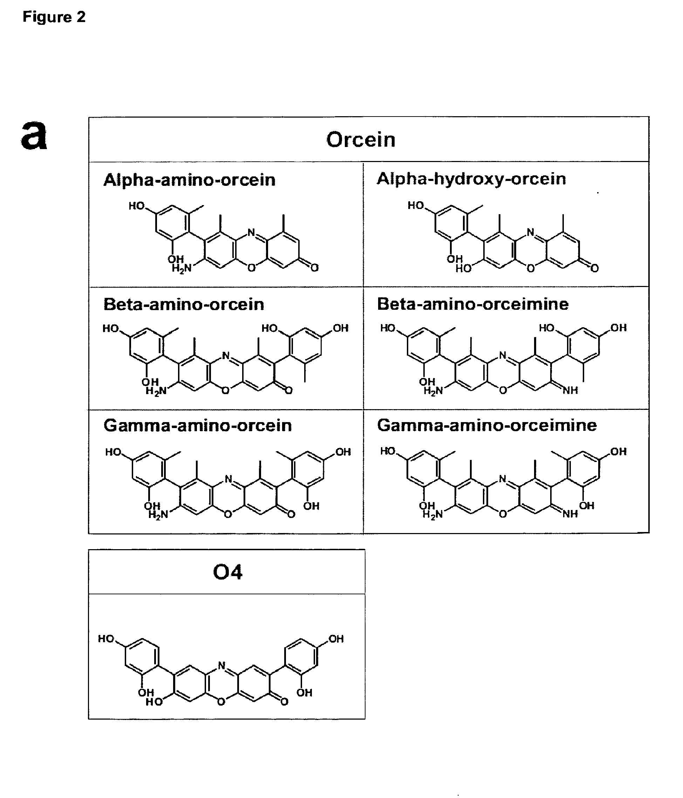

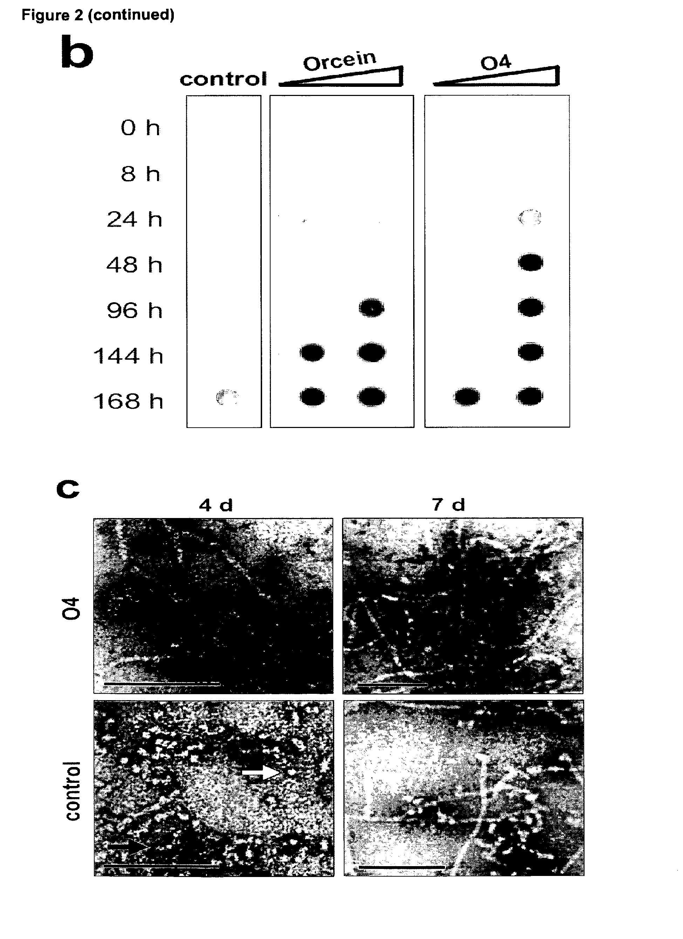

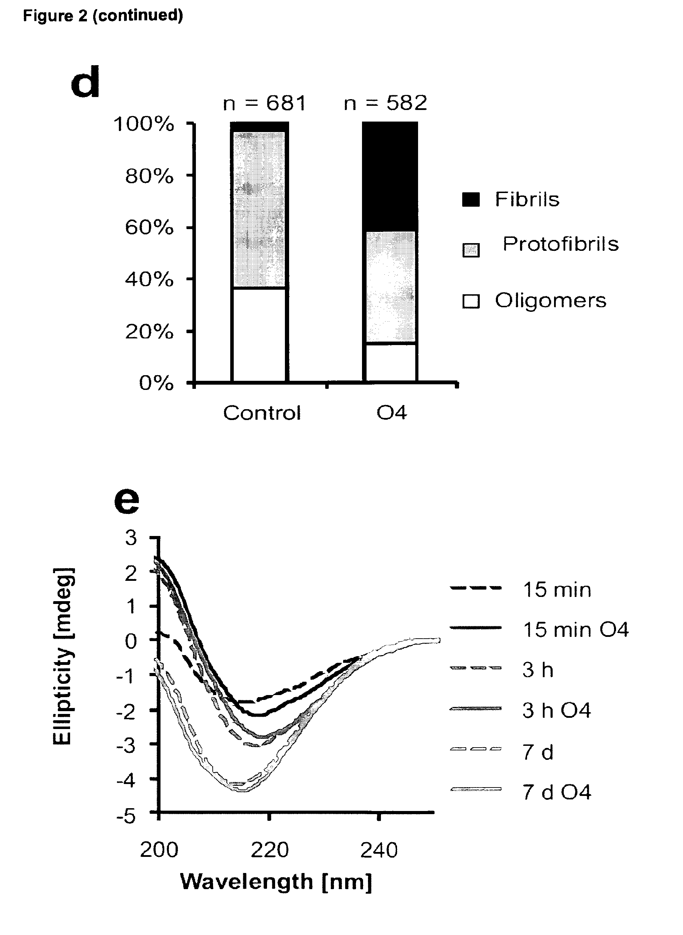

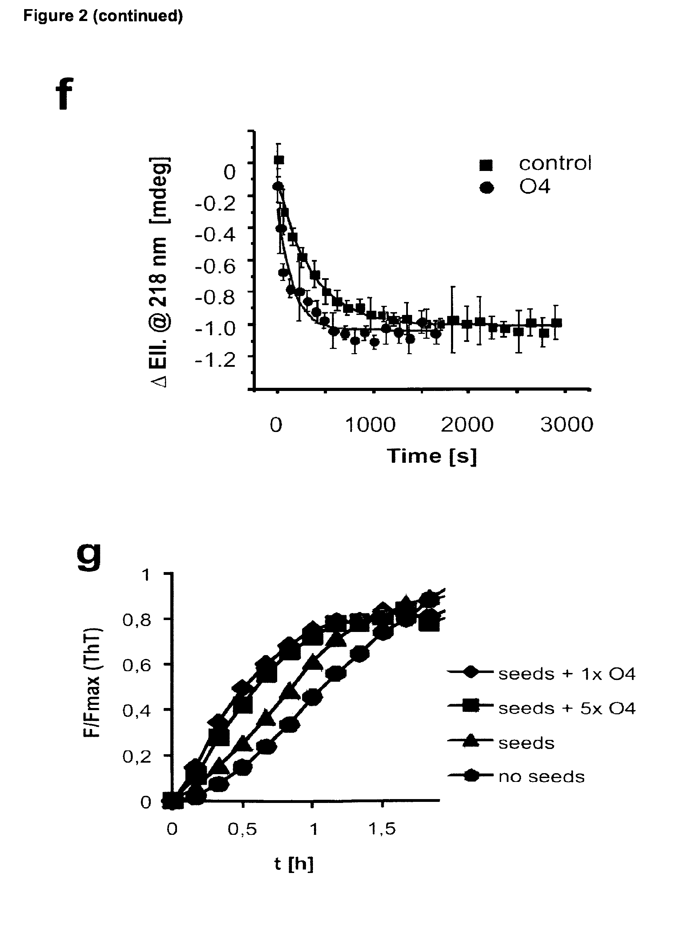

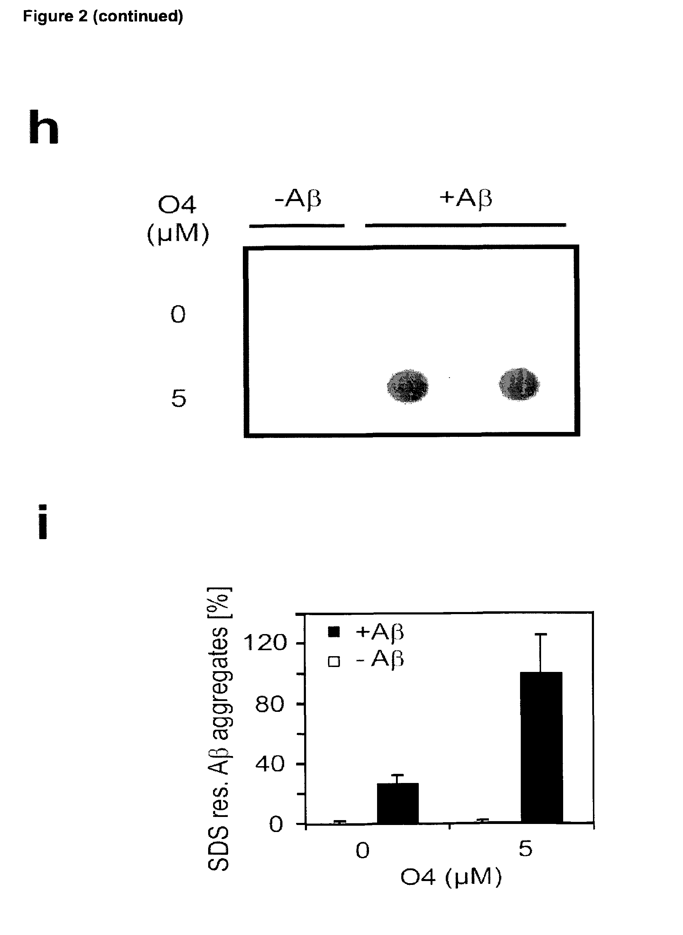

**Fig. 2****:** Orcein and 04 accelerate the formation of SDS-resistant Aβ₄₂ aggregates *in vitro*. **a,** Structural formulae of orcein and 04. Orcein is a compound mixture consisting of six main components. **b,** Effects of orcein or 04 on Aβ₄₂ aggregate formation. Spontaneous assembly of SDS-stable Aβ₄₂ aggregates was quantified by FRA. Aβ₄₂ (15 µM) was incubated with an equimolar or 5-fold molar excess of chemical compounds; SDS-stable aggregates were detected using the monoclonal anti-Aβ antibody 6E10. **c,** Analysis of Aβ₄₂ aggregation by transmission electron microscopy (TEM). Aβ₄₂ (15 µM) was incubated in the presence and absence of 04 (150 µM) and samples were analysed after incubation for 4 and 7 d. Bars indicate a length of 100 nm. Arrows indicate oligomers (white) or protofibrils (black). **d,** Quantitative analysis of different types of Aβ₄₂ aggregate species produced in the presence and absence of 04 (150 µM). The number of fibrils, protofibrils and oligomers was quantified after incubation for 48 h. **e,** Analysis of Aβ₄₂ aggregates by CD spectroscopy. Aβ₄₂ (15 µM) peptides were incubated at 37°C with an equimolar concentration of 04 in phosphate buffer. 04 induced Aβ₄₂ fibrils have a typical β-sheet structure. **f,** 04 (75 µM) accelerates β-sheet formation of monomeric Aβ₄₂ (15 µM). Peptides were incubated with 04 at 37°C in phosphate buffer and β-sheet formation was monitored by the change in ellipticity at 218 nm. CD data were fitted by monoexponential decay functions with time constants of τ = 350 ± 30 nm (control) and τ = 150 ± 40 nm (O4). **g,** 04-generated Aβ₄₂ fibrils are seeding-competent structures. Aβ₄₂ fibrils were produced *in vitro* with an equimolar (1x) or 5-fold molar excess of 04 and added after sonification (3 % v/v) to Aβ₄₂ (15 µM) monomers. Formation of amyloid fibrils was monitored by Thioflavin T (ThT) fluorescence assay. h, Treatment of mammalian cells with 04 stimulated the formation of SDS-resistant Aβ. aggregates. 7PA2 cells were transfected with preformed Aβ₄₂ aggregates (final concentration 0.6 µM) and incubated for 2 d with 5 µM 04. SDS-stable aggregates were detected by FRA using the monoclonal anti-Aβ, antibody 6E10. i, Relative quantification of SDS-resistant Aβ₄₂ aggregates, n = 4, means ± SD.

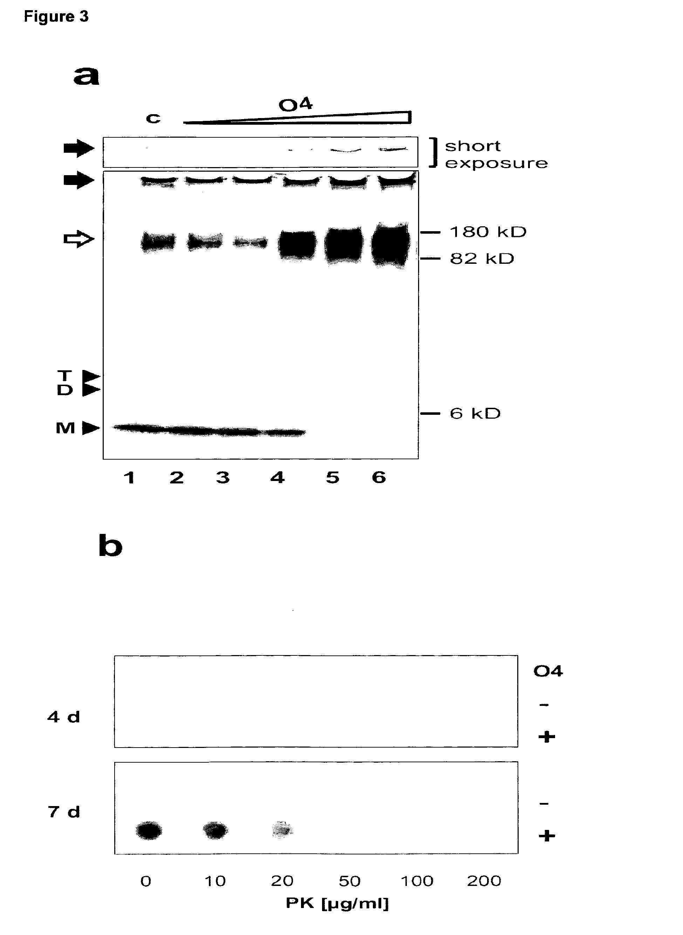

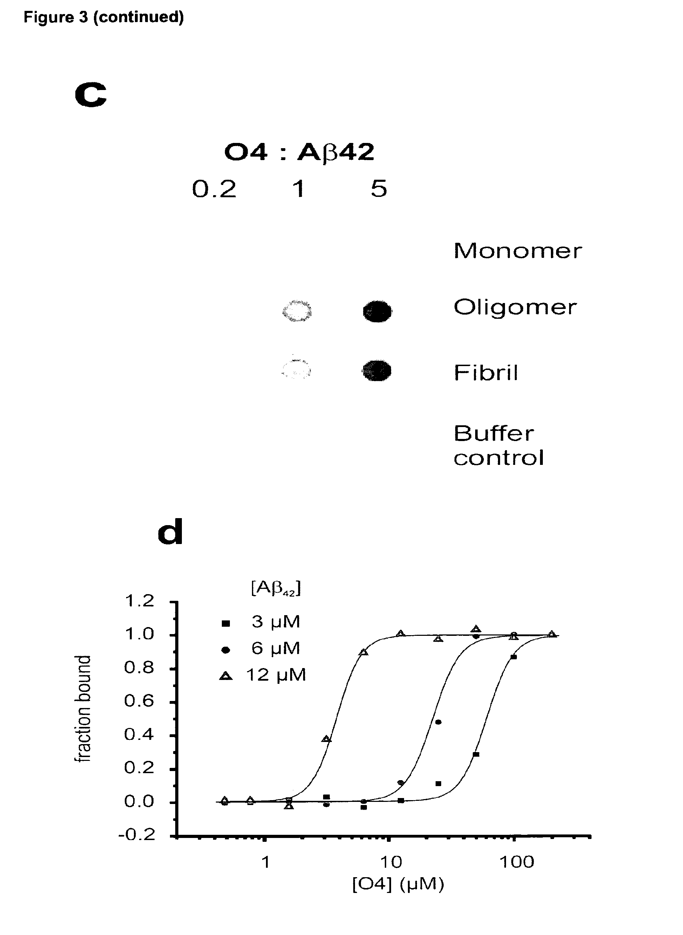

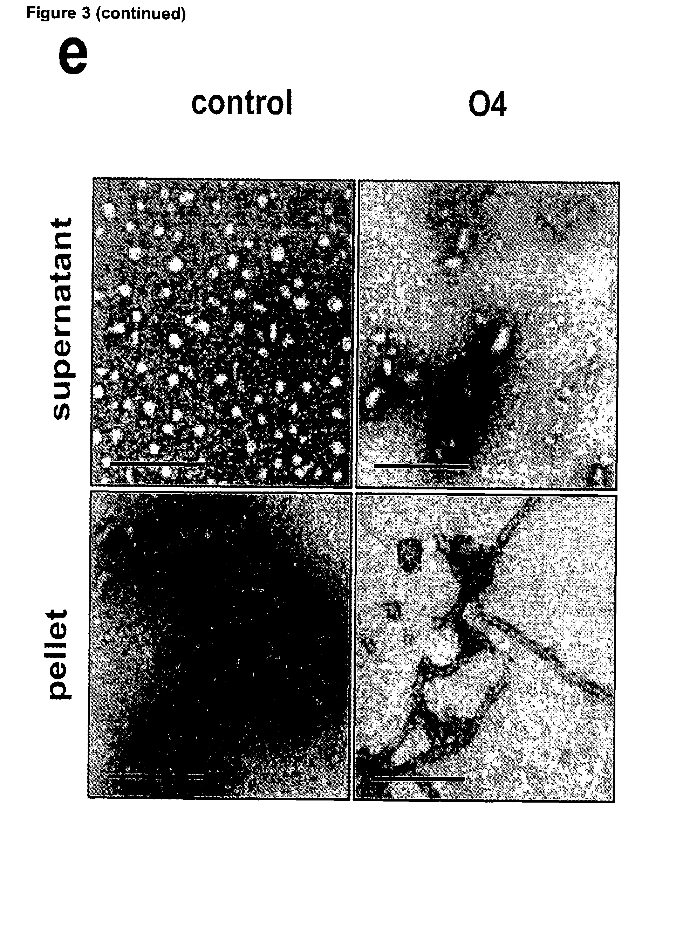

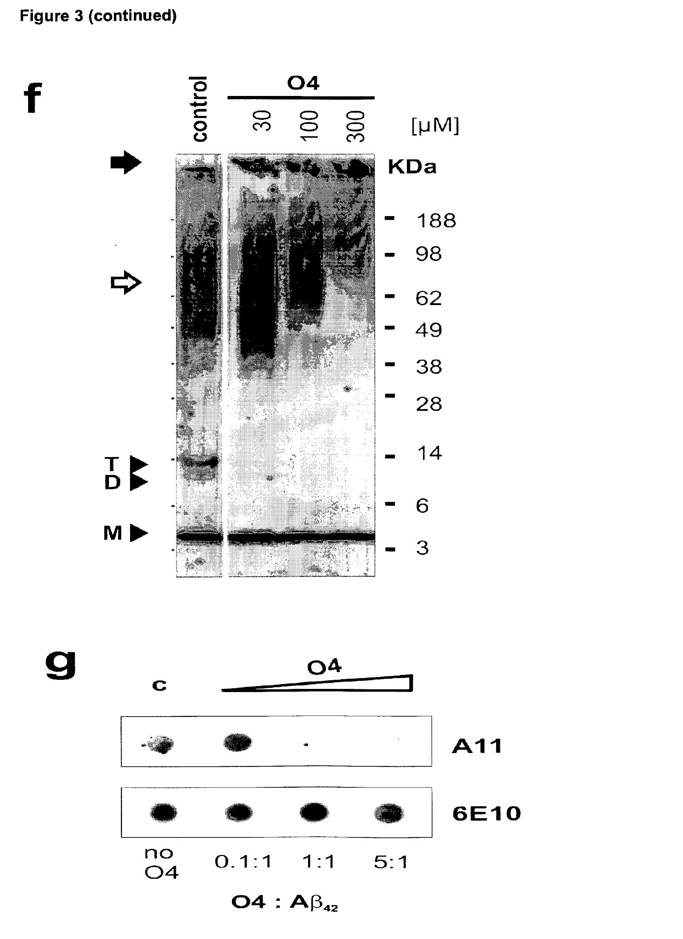

**Fig. 3****:** O4 binds to Aβ₄₂ oligomers and stimulates their conversion into larger SDS-stable amyloid fibrils. **a,** Analysis of 04-generated Aβ₄₂ aggregate species under denaturating conditions. Aβ₄₂ peptides (15 µM) were incubated with different concentrations of 04 in phosphate buffer at 37°C and samples were analyzed after 48 h by SDS-PAGE and silver staining. 1, control without 04; 2, 1.5 µM 04; 3, 7.5 µM 04; 4, 15 µM 04; 5, 75 µM 04; 6, 150 µM 04. M, monomer; D, dimer; T, trimer; open arrow, medium-size oligomers; filled arrows, large SDS-insoluble aggregates. 04 increases the levels of SDS-insoluble Aβ₄₂ aggregates in a concentration-dependent manner. **b,** Analysis of SDS-stable Aβ₄₂ aggregates after proteinase K treatment. Aβ₄₂ peptides (15 µM) incubated in the presence and absence of 04 (15 µM) for 4 and 7 d at 37°C in phosphate buffer were treated for 30 min with different concentrations of proteinase K (10-200 µg/ml) and subsequently analyzed by FRA using the 6E10 antibody. 04 stimulates the assembly of SDS-stable, proteinase K-resistant protein aggregates. **c,** 04 binds to Aβ₄₂ oligomers and fibrils but not to monomers. Preparations with monomeric, oligomeric and fibrillar Aβ₄₂ (15 µM) were incubated with 04 (3, 15 and 75 µM) for 15 min and compound binding was analyzed colorimetrically after dot blotting of samples onto a nitrocellulose membrane. **d,** Analysis of 04 binding to Aβ₄₂ oligomers. 04 (0.4-200 µM) binding assays were performed as in c, 04 binding was evaluated densitometrically. 04 signals under saturating conditions were set to 100% and plotted against 04 concentration on a logarithmic scale. Lines represent fitting by a cooperative dose-response curve y = 1/ (1+10^(LogEC50- log [O4])*n) with a Hill coefficient of n = 4. **e,** Analysis of Aβ₄₂ oligomerization in the presence and absence of 04 by TEM. Aβ₄₂ peptides (100 µM) were incubated with 04 (10-fold molar excess) for 24 h at 6-8°C in F12 medium and soluble aggregation products (oligomers) were subsequently separated from insoluble material (protofibrils and fibrils) by centrifugation. 04 treatment reduced the levels of transient oligomers by accelerating fibrillogenesis. Size bars represent 100 nm. **f,** Analysis of 04 treated oligomer preparations by SDS-PAGE and immunoblotting. Preformed Aβ₄₂ oligomers (110 µM) were incubated for 24 h with different concentrations of 04 (30, 100 and 300 µM) and subsequently analyzed by SDS-PAGE and immunoblotting using the monoclonal anti-Aβ antibody 57-11-4. **g,** 04 treatment reduces the formation of A11-reactive amyloid oligomers in cell-free aggregation reactions. Aβ₄₂ peptides (15 µM) were incubated for 48 h at 37°C in the presence and absence of 04; formation of amyloid oligomers was detected by dot blot assays using the A11 antibody. Total Aβ₄₂ levels were detected with the 6E10 antibody.

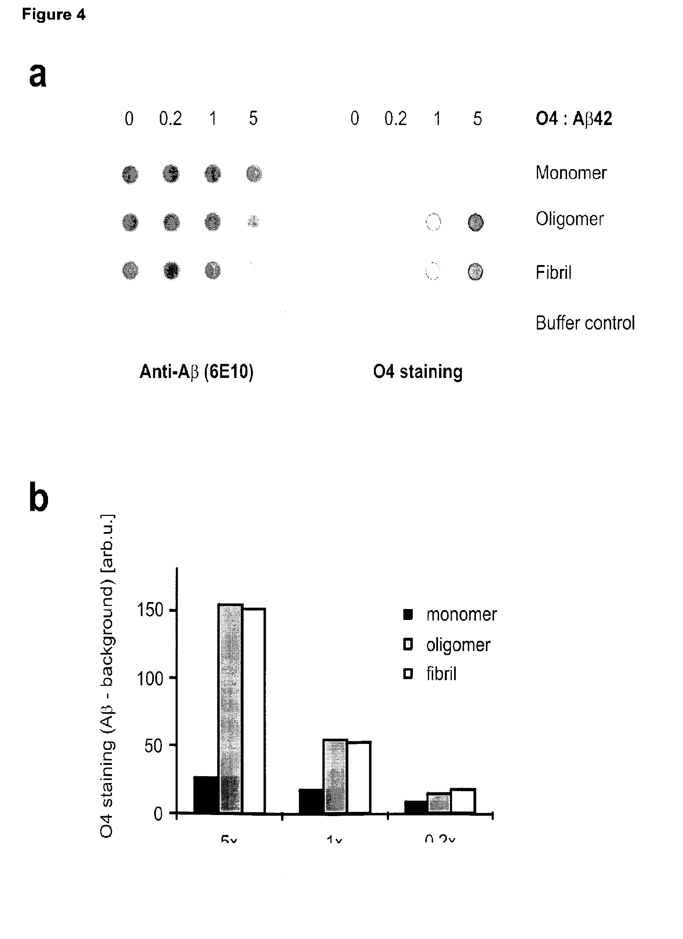

**Fig. 4****:** O4 binds to Aβ₄₂ oligomers and fibrils but not to monomers. Aβ₄₂ oligomers (110 µM) were produced in vitro (37°C for 24 h) and centrifuged (14,000 rpm), fibrils were produced by incubation of monomeric Aß₄₂ (15µM) for 7 d at 37°C in PBS. a, Monomeric, oligomeric and fibrillar Aß₄₂ (15 µM) preparations were incubated with 04 (3, 15 and 75 µM) for 15 min; proteins were dot blotted onto a nitrocellulose membrane and the membrane was subsequently washed over night in PBS (+ 0.1 % Tween20 + 0.1% Triton X-100). 04 binding was analyzed colorimetrically. Aß₄₂ peptides spotted onto the membrane were quantified by immunostaining using the 6E10 antibody. b, Quantification of relative 04 signals. 04 unspecifically bound to the membrane (buffer control) was subtracted from Aß₄₂ -04 signals. The average difference in 04 signal between duplicate samples was ∼10%.

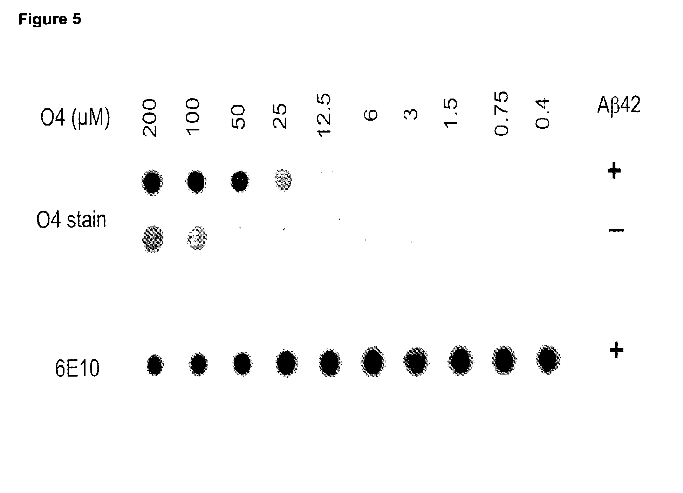

**Fig. 5****:** Quantification of 04 binding to Aβ₄₂ oligomers. Preparations with oligomeric Aβ₄₂ (6 µM) were incubated with 04 (0.4-200 µM) for 15 min at RT. Samples were blotted onto a nitrocellulose membrane and membranes were washed over night with PBS + 0.05% Tween 20+0.1% Triton X-100. Compound binding to oligomers was quantified densitometrically. Aβ₄₂ peptides bound to filter membranes were subsequently also detected by immunostaining using the 6E10 antibody. Equivalent amounts of 04 in the absence of Aβ₄₂ peptides were blotted in control samples and unspecific membrane staining was subtracted for quantitative binding analysis.

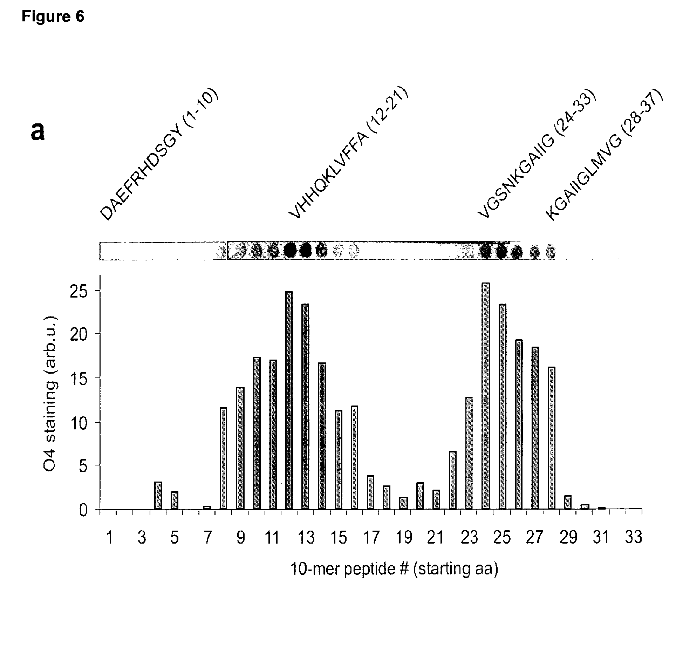

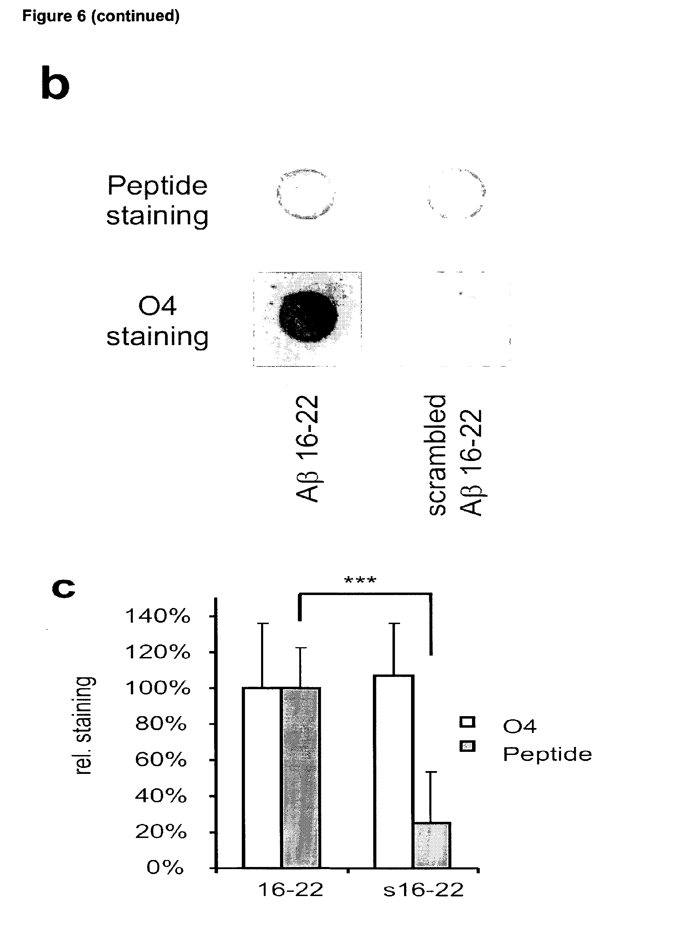

**Fig. 6****:** O4 binds to hydrophobic regions in Aβ₄₂ peptides. **a,** Incubation of a 10-mer peptide array with 04 (300 µM). Peptide numbers represent the starting amino acid of 10-mer peptides. 04 binding to peptides was quantified colorimetrically. To potential 04 binding regions in Aβ₄₂ peptides were detected (peptides 8-16 and 23-28). **b,** Dot blot analysis of 04 binding to Aβ 16-22 and a scrambled version of this peptide. 04 binding to peptides was detected colorimetrically; in control experiments peptides were also stained with the unspecific dye amide black. c, Quantification of relative 04 and amide black staining; n = 4.

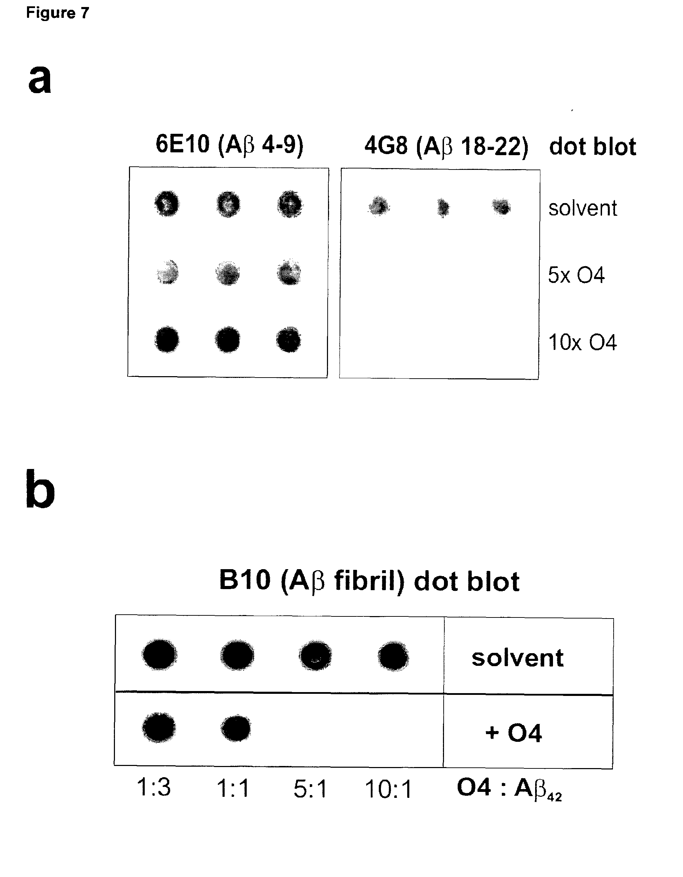

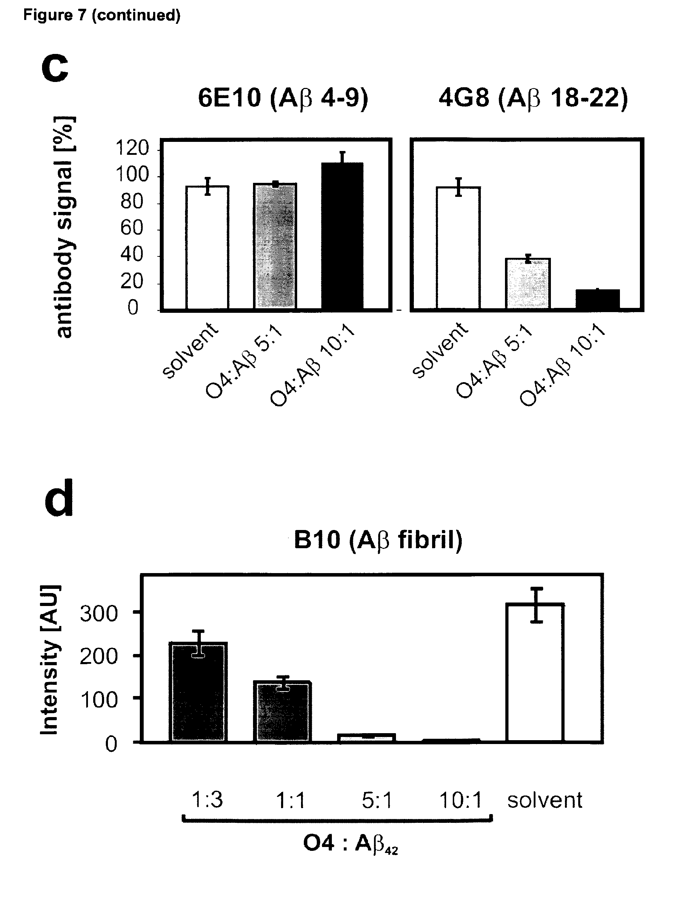

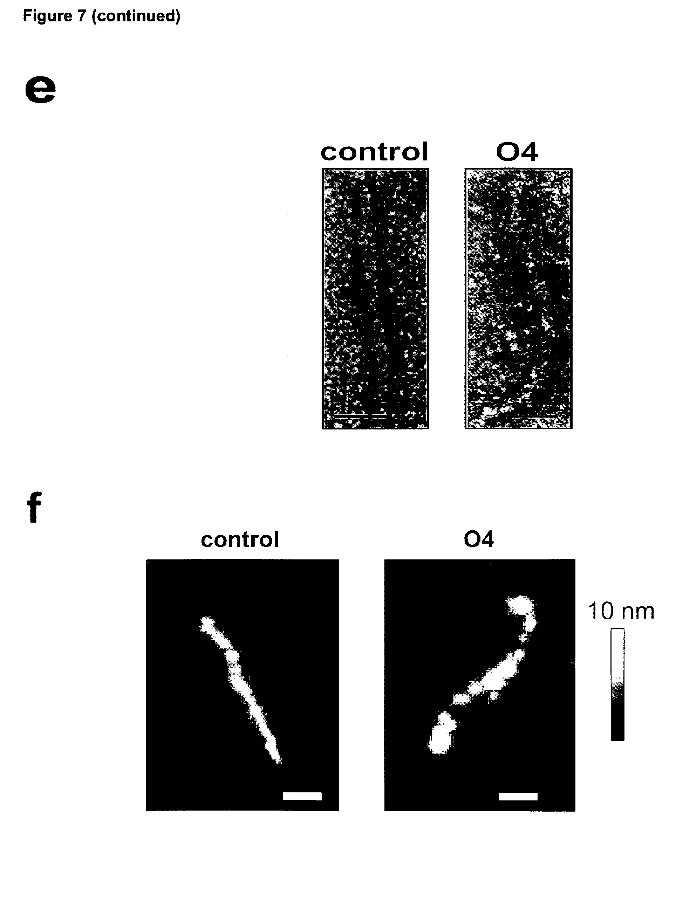

**Fig. 7****:** O4 binds to Aβ₄₂ amyloid fibrils and changes their surface properties. **a,** Dot blot assays of 04 treated and untreated Aβ₄₂ (15 µM) aggregation reactions (5 d at 37°C). Aggregates were detected using the epitope-specific monoclonal anti-Aβ antibodies 6E10 (aa 4-9) and 4G8 (aa 18-22), respectively. 04-treatment reduces the binding of 4G8 but not of 6E10 to amyloid fibrils. **b,** Dot-blot assays of 04 generated Aβ₄₂ aggregates with the fibril-specific antibody B10. Aβ₄₂ peptides (15 µM) were incubated for 48 h at 37°C with different concentrations of 04 and subsequently analyzed by dot blotting. 04 treatment reduces B10 binding to amyloid fibrils in a concentration-dependent manner. **c,d,** Bar graph quantification of triplicate samples ± SD from a and b. **e, f,** Analysis of 04-generated amlyloid fibrils by TEM and AFM. 04-generated amyloid fibrils (15 µM Aβ₄₂, 4 d non-agitated incubation, 37°C, 75 µM 04) are morphologically different from untreated Aβ ₄₂ fibrils; scale bars 100 nm.

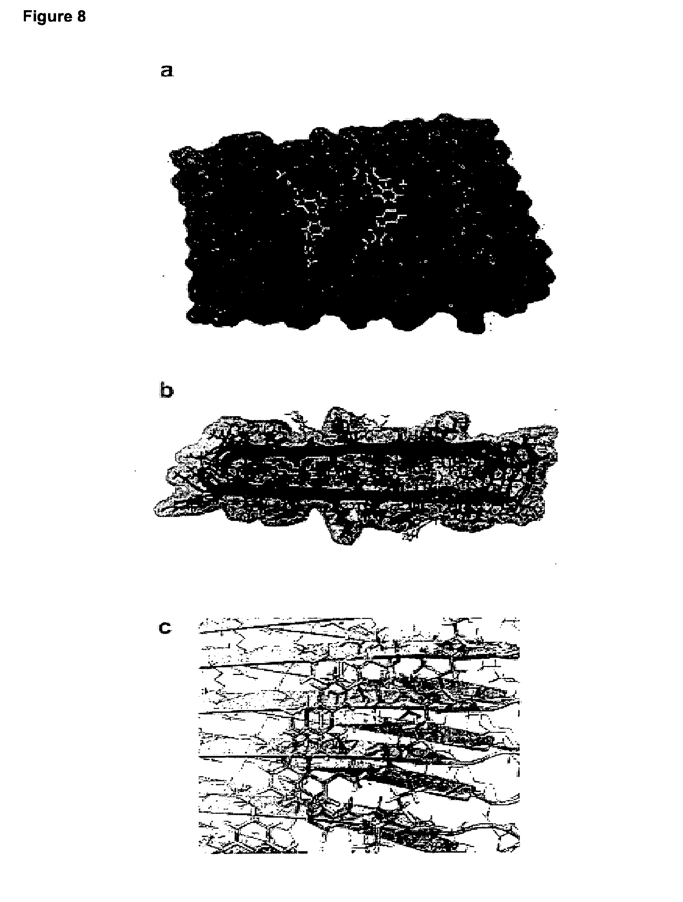

**Fig. 8****:** O4 binds to hydrophobic grooves on the surface of β-sheet-rich Aβ₄₂ protofibrils. **a,** Computational docking studies predict that 04 binds parallel to the long fibril axis targeting hydrophobic grooves at aa 33-35, 35-37 or 20-21. **b,** Side view of an Aβ₄₂ fibril with bound 04 molecules. **c,** Predicted interaction of the oxazine ring in 04 with the aromatic rings of phenylalanine 20 (F20) in Aβ₄₂ protofibrils.

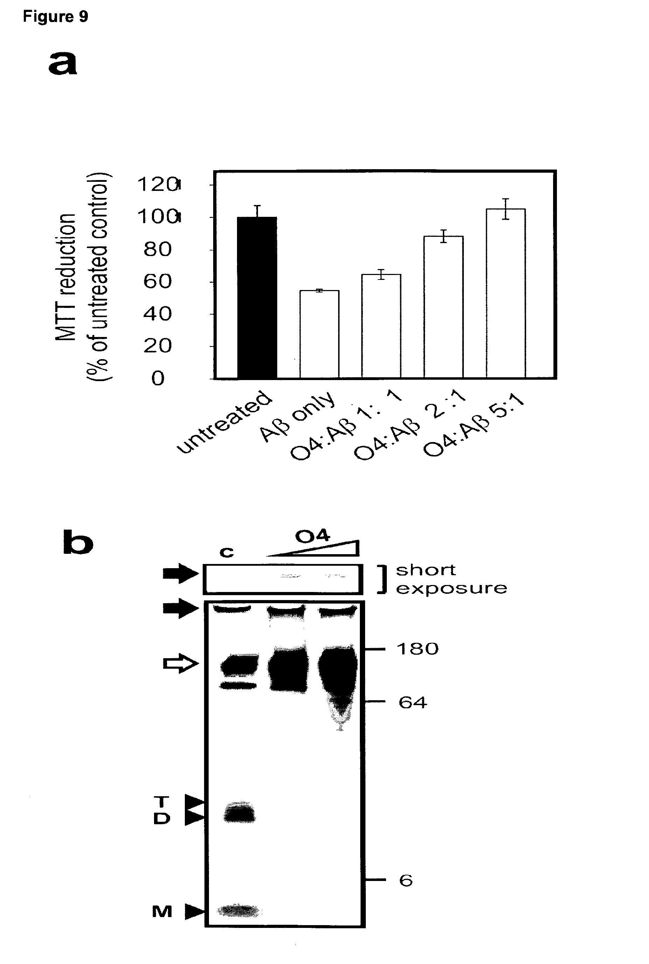

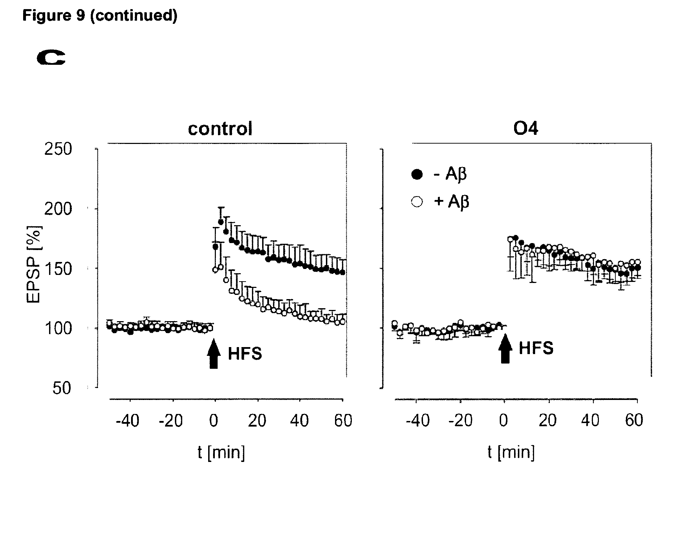

**Fig. 9****:** O4-mediated acceleration of Aβ₄₂ fibrillogenesis correlates with reduced cellular toxicity and improves synaptic transmission in brain slices. **a,** 04 treatment reduces Aβ₄₂ toxicity in a SHSY5Y cell model. Aβ₄₂ peptides (5 µM) and 04 were added to the culture medium of differentiated SHSY5Y cells and toxicity was monitored after an incubation period of 48 h using a standardized MTT assay. 04 treatment significantly improved cell viability in a concentration-dependent manner. **b,** Addition of 04 (5 µM or 25 µM) to SHSY5Y cell cultures promotes Aβ₄₂ (5 µM) aggregation reducing the levels of soluble monomers and small oligomers. Samples were incubated for 48 h and analyzed by SDS-PAGE and silver staining. **c,** 04 treatment reduces Aβ₄₂-induced inhibition of long-term potentiation (LTP) in hippocampal rat brain slices. LTP of excitatory post-synaptic potentials (EPSP) was evoked by high-frequency stimulation (HFS).

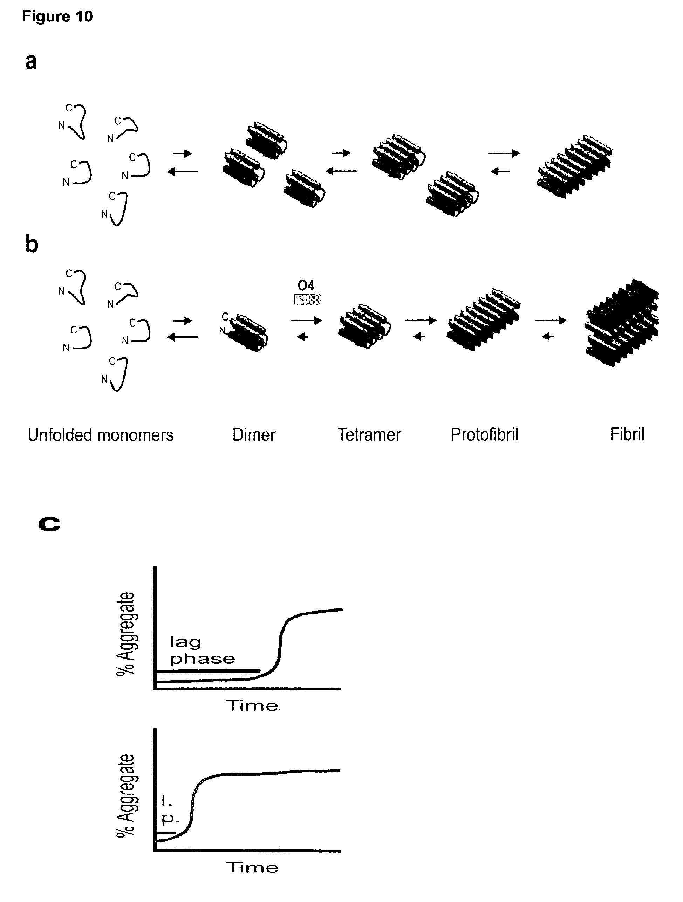

**Fig. 10****:** Working model of effects of 04 on spontaneous Aβ₄₂ polymerization. a, Untreated, spontaneous Aβ₄₂ aggregation reaction. Seeding-competent, ß-sheet-rich Aβ₄₂ aggregates (protofibrils) are slowly formed during the nucleation (lag) phase by association of monomers and/or unstructured oligomers. b, In 04 treated reactions formation of amyloid fibrils is accelerated because compound binding to small aggregates stabilizes the formation of low molecular weight, ß-sheet-rich prefibrillar structures (tetramers, protofibrils etc.) that polymerize efficiently into mature amyloid fibrils. Thus, 04 treatment of heterogeneous Aβ₄₂ aggregation reactions alters the kinetics of amyloid polymerization leading to a reduced lag phase followed by a rapid exponential fibril growth phase. c, 04 treatment reduces the lag phase of Aβ₄₂ aggregation reactions.

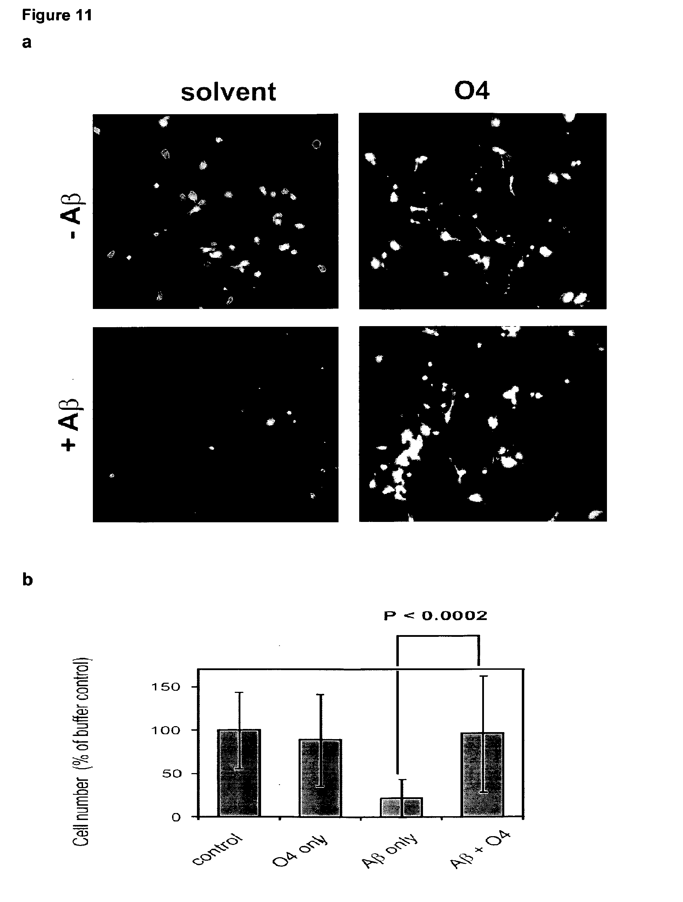

**Fig. 11****:** Working model of effects of 04 on spontaneous Aβ₄₂ polymerization. **a,** Untreated, spontaneous Aβ₄₂ aggregation reaction. Seeding-competent, β-sheet-rich Aβ₄₂ aggregates (protofibrils) are slowly formed during the nucleation (lag) phase by association of monomers and/or unstructured oligomers. **b,** In 04 treated reactions formation of amyloid fibrils is accelerated because compound binding to small aggregates stabilizes the formation of low molecular weight, b-sheet-rich prefibrillar structures (tetramers, protofibrils etc.) that polymerize efficiently into mature amyloid fibrils. Thus, 04 treatment of heterogenous Aβ₄₂ aggregation reactions alters the kinetics of amyloid polymerization leading to a reduced lag phase followed by a rapid exponential fibril growth phase.

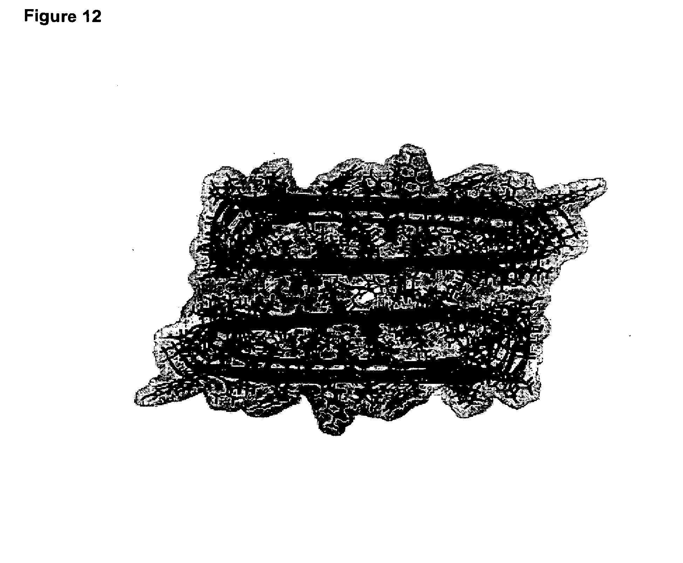

**Fig. 12****:** Docking model of 04 molecule bridging two strands of an Aβ₄₂ fibril. The 04 molecule interacts with the hydrophobic grooves at G33-M35 of both Aβ strands.

### Examples

### 1. Synthesis of Compound 04

Compound 04 may be synthesized as follows: 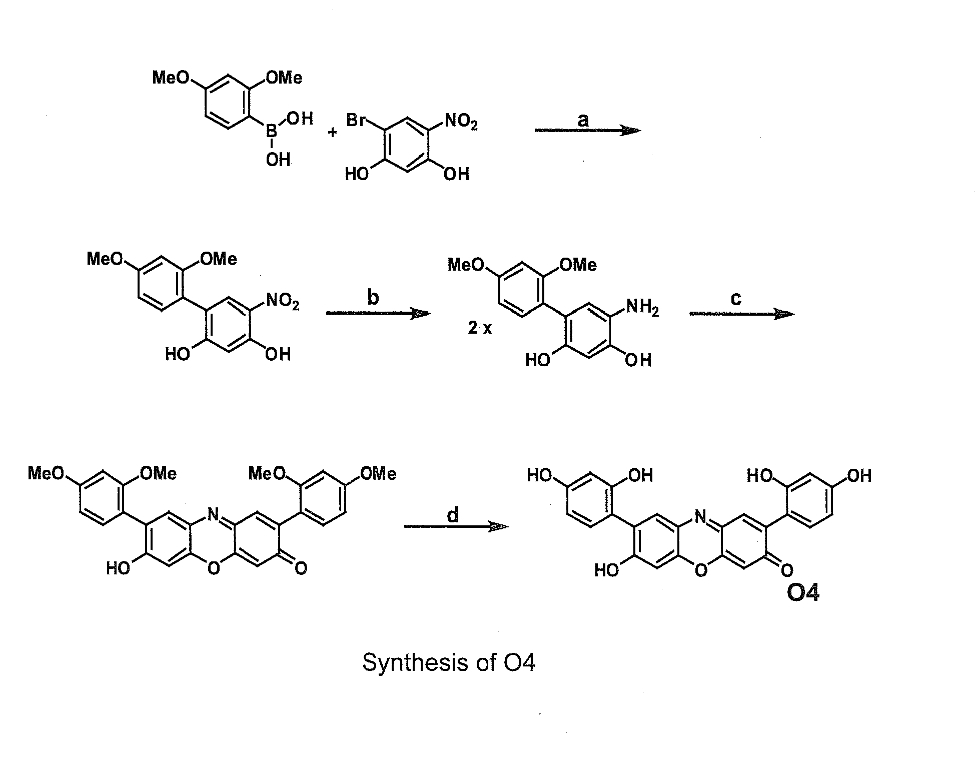
a) 2,4-dimethoxyphenylboronic acid (2.0 g) + 4-bromo-6-nitrobenzene-1,3-diol (2.57 g), palladium(II) acetate (246 mg), triphenylphosphine (1,152 g), K2C03 (1.517 g), dioxane : water 6:1, 16 h, 80°C aqueous workup, chromatography, yield: 115 mg;
b) 2',4'-dimethoxy-5-nitrobiphenyl-2,4-diol (300 mg), Pd/C (90 mg), ethyl acetate:ethanol 5:4, 4 h, 20°C, filtration, yield: 220 mg;
c) 5-amino-2',4'-dimethoxybiphenyl-2,4-diol (500 mg) + lead(IV) acetate (1.6 g), glacial acetic acid, 30 min, aqueous workup, yield: 60 mg;
d) 3H-phenoxazin-3-one (80 mg) + BBr3 (95 µL), dichloromethane, 18 h, -80°C → 25°C aqueous workup, chromatography, yield: 20 mg, 1H-NMR (300 MHz, d6-DMSO) ppm: 6.16-6.18 (m, 2H), 6.23 (d, 2H, J = 2.4 Hz), 6.31 (dd, 2H,J = 2.4 Hz, J = 8.4 Hz), 6.87-6.93 (m, 2H), 7.16 (d, 2H, J = 8.5 Hz), 7.49 (s, 1H),9.3(bs,4H).

### 2. Inhibition of amyloid formation

### 2.1. Materials and Methods

**2.1.1. Protein aggregation.** For aggregation reactions, Aβ₄₂ stock solution (1 mM in DMSO) was diluted in phosphate buffer (100 mM sodium phosphate, 10 mM NaCl, pH 7.4) to 15 µM and incubated in 1.5 ml Eppendorf tubes (Eppendorf, Germany) at 37°C. Generation of soluble Aβ₄₂ oligomers was performed as described (Lambert, 1998). Aβ₄₂ DMSO stock solutions were diluted in Ham's F12 medium without phenol red (Biosource) at a final peptide concentration of 100 µM, with or without test compounds. After brief vortexing, samples were incubated at 4-6°C for 24 h. Alternatively, Aβ₄₂ oligomers were prepared as described in (Ma, 2006) by diluting DMSO stock solution to a final Aβ₄₂ concentration of 110 µM in 10 mM HEPES buffer pH 7.4. Samples were incubated in a glass vial with a micro stir bar at 37°C for 24 h and subsequently centrifuged (Beckman Coulter Microfuge R, rotor F241.5P) at 13,000 rpm.

**2.1.2. Filter retardation assay (FRA).** This assay was performed as described previously (Wanker, 1999). Briefly, 60 µl of Aβ₄₂ aggregation reactions were added to an equal volume of 4 % sodium dodecyl sulfate (SDS) solution containing 100 mM dithiothreitol (DTT) and boiled at 98°C for 5 min. 85 µl of the heat denatured samples were filtered through a cellulose acetate membrane with 0.2 µm pores (OE66, Schleicher and Schuell, Germany). Membranes were blocked in Tris-buffered saline containing 3 % skim milk. Aggregates retained on the filter membrane were detected using the 6E10 antibody (1 : 5.000) (Sigma, Deisenhofen, Germany), and secondary antibodies conjugated to either horseradish peroxidase or alkaline phosphatase (Promega, Germany). Proteinase K (final concentration of 10-200 µg/ml) was added to Aβ₄₂ aggregation reactions after 4 or 7 d incubation at 37°C. After 30 min incubation at 37°C with proteinase K, samples were denatured and analyzed by FRA.

### 2.2. Results

**2.2.1 Identification of small molecules that stimulate Aβ₄₂ aggregation.** In order to find chemical compounds that promote Aβ₄₂ amyloidogenesis, a membrane filter retardation assay (FRA) was established that allows the identification of large, insoluble protein aggregates, while monomers and small, soluble oligomers are not detected (Fig. 1). Using this method, we screened a focused library of -300 chemical compounds that were previously identified as modulators of polyglutamine-mediated huntingtin exon1 aggregation (Heiser, 2002). We found that the natural dye orcein very efficiently promotes Aβ₄₂ aggregation in vitro (Fig. 2a and b).

Orcein is not a defined chemical compound, but rather a mixture of closely related substances such as α-, β- and γ-amino-orcein (Beecken, 2003). In order to identify a pure compound that stimulates Aβ₄₂ aggregation, several chemical compounds were tested and the substance 2,8-bis-(2,4-dihydroxyphenyl)-7-hydroxy-phenoxazin-3-one, termed 04, was identified as a potent Aβ₄₂ aggregation accelerator. 04 is structurally similar to components in the orcein mixture (Fig. 2a) and promotes spontaneous assembly of Aβ₄₂ insoluble aggregates in a concentration-dependent manner (Fig. 2b).

**2.2.2. 04 promotes the assembly of seeding-competent, β-sheet-rich Aβ₄₂ fibrils.** To examine the morphology of 04-generated protein aggregates, time course experiments were carried out and evaluated by transmission electron microscopy (TEM). After an incubation period of 4 d, worm-like Aβ₄₂ protofibrils (Walsh, 1999) with a length of ∼30-150 nm and a diameter of -5 nm were predominantly detected in untreated aggregation reactions (Fig. 2c). In contrast, mostly longer amyloid fibrils (>500 nm) were observed in O4-treated samples, indicating accelerated amyloid formation. A similar result was obtained after an incubation period of 7 d (Fig. 2c). A quantitative analysis of aggregate species (fibrils, protofribrils and oligomers) after 48 h confirmed these results (Fig. 2d). The number of long amyloid fibrils (27%) was significantly larger (p < 1*10⁻⁵) in 04 treated samples than in untreated control samples (1.3%). At the same time, the number of oligomeric structures was reduced in 04-treated aggregation reactions (p < 1*10⁻⁴)_{.}

Previous studies have demonstrated that insoluble Aβ₄₂ fibrils have a characteristic cross-β-sheet conformation (Dobson, 2003). Therefore, we investigated whether 04 treatment influences the structure of Aβ₄₂ amyloid fibrils using circular dichroism (CD) spectroscopy (Ehrnhoefer, 2008). We found that both 04-treated and untreated Aβ₄₂ fibrils have CD spectra characteristic of β-sheet-rich amyloid structures (Fig. 2e), indicating that compound-mediated acceleration of Aβ₄₂ aggregation does not substantially alter the conformation of amyloid fibrils.

Next, the conversion of unstructured Aβ₄₂ monomers into β-sheet-rich early aggregates was investigated. The change in CD signal at 218 nm over the course of 3,000 s was monitored for 04-treated and untreated Aβ₄₂ peptides.

A monoexponential data fit yielded a time constant of τ = 150 ± 40 s for O4-treated samples, while a time constant of τ = 350 ± 30 s was obtained for untreated samples. This suggests that 04 significantly accelerates the transition of unstructured Aβ₄₂ molecules into larger β-sheet-rich aggregate assemblies in cell-free assays (Fig. 2f).

We also investigated whether 04-generated Aβ₄₂ fibrils are indeed seeding-competent structures. Amyloid fibrils were produced *in vitro* in the presence and absence of 04 and added as seeds to an excess of unpolymerized Aβ₄₂ monomers. The formation of β-sheet-rich amyloid fibrils was then monitored over time using a Thioflavin T (ThT) dye binding assay (LeVine, 1999). We found that 04-generated Aβ₄₂ seeds as well as untreated seeds both accelerate amyloid polymerization in cell-free assays (Fig. 2g). Thus, the β-sheet-rich Aβ₄₂ fibrils produced in the presence of 04 are seeding-competent structures that efficiently promote amyloid fibrillogenesis.

The effect of 04 on template-mediated Aβ₄₂ aggregation was also confirmed using a cell-based assay. CHO cells overexpressing mutant human APP protein (Walsh, 2002) were transfected with preformed Aβ₄₂ fibrils (Yang, 2002; Bieschke 2010) and subsequently treated for 48 h with 5 µM 04. Then, the formation of SDS-stable, insoluble Aβ aggregates was monitored by FRA using an anti-amyloid-β antibody. A -4-fold higher amount of insoluble Aβ aggregates was detected in 04 treated than in untreated samples (Fig. 2 h and i), indicating that the compound stimulates amyloid polymerization in mammalian cells.

**2.2.3. O4 promotes the formation of SDS-stable, protease-resistant protein aggregates.** To investigate whether 04 treatment influences the stability of amyloid structures, we analyzed compound-treated Aβ₄₂ aggregation reactions after 48 h with SDS-PAGE and silver staining (Fig. 3a). In the absence of 04, aggregation products resolved to monomers (-4 kDa), small oligomers (-10-12 and 14-16 kDa), medium-size oligomers (-100-150 kDa) and large aggregates (>250 kDa) in SDS gels, supporting previously published studies that Aβ₄₂ monomers *in vitro* assemble into a heterogenous mixture of low and high molecular weight aggregate species (Bitan, 2003). However, in the presence of 04 an altered distribution of SDS-stable Aβ₄₂ aggregate species was observed. We found that in 04 treated samples the relative amount of medium-size, SDS-stable oligomers and large aggregates was increased, while the amount of monomers and small oligomers was decreased (Fig. 3a). This indicates that 04 treatment results in the formation of highly stable fibrillar structures (Fig. 2c and d) that, under denaturating conditions, do not disassemble into monomers and low molecular weight oligomers.

Then, it was also investigated whether 04 treatment influences the protease resistance of Aβ₄₂ fibrils. Aggregates generated in the presence or absence of 04 (4 and 7 d at 37°C) were treated with different concentrations of proteinase K (30 min at 37°C) and subsequently analyzed by FRA. We observed that 04-generated Aβ₄₂ aggregates are more proteinase K resistant than untreated aggregates (Fig. 3b), supporting the results from the SDS-PAGE and silver staining experiments (Fig. 3a).

**2.2.4. 04 converts small, unstable Aβ₄₂ oligomers into larger SDS-stable protein aggregates.** To examine whether 04 converts small Aβ₄₂ oligomers into larger amyloid fibrils, we first tested whether the compound can bind to different types of preformed aggregate species. Peptide preparations highly enriched in Aβ₄₂ monomers (Ehrnhoefer, 2008), spherical oligomers (Ma, 2006) or large amyloid fibrils (Ehrnhoefer, 2008) were produced and incubated with 04 for 15 minutes at room temperature. Then, samples were spotted onto a nitrocellulose membrane and after washing with buffer (non-denaturing conditions) 04 binding was examined. As 04 is a dye and blue in color its binding to amyloid aggregates can be detected colorimetrically (Fig. 3c). We found that 04 readily stains oligomeric and fibrillar Aβ₄₂ aggregates, while monomers were less efficiently detected, indicating that the compound preferentially associates with on-pathway, β-sheet-rich amyloid assemblies. Control membranes dot-blotted with identical Aβ₄₂ peptide fractions were analyzed using the monoclonal anti-Aβ antibody 6E10 (Fig. 4), demonstrating that comparable amounts of the Aβ₄₂ preparations were spotted onto membranes.

To assess the affinity of 04 binding to Aβ₄₂ oligomers, the compound (0.4 -200 µM) was incubated for 15 min with preformed aggregate species (equivalent to monomer concentrations of 3, 6 and 12 µM) and samples were subsequently spotted onto nitrocellulose membranes. Binding of 04 to Aβ₄₂ oligomers was quantified colorimetrically (Fig. 5). Quantitative analysis of binding data revealed apparent dissociation constants of 50±2 µM, 25±1 µM, and 3.5±0.1 µM, respectively (Fig. 3d), indicating that binding data cannot be fitted in a satisfactory manner using a standard single site binding model. When a cooperative binding model with a Hill coefficient of n = 4 was used, however, a reasonable representation of the data was possible, suggesting that 04 binds to oligomers by a cooperative mechanism requiring 4 Aβ₄₂ molecules for efficient association.

We then investigated whether 04 treatment influences the assembly of small Aβ₄₂ oligomers, which are preferentially formed at low temperature in cell-free assays (Lambert, 1998). Aβ₄₂ monomers were incubated for 24 h at 6-8°C in the presence and absence of 04 and formation of oligomers was analyzed by TEM. In agreement with previously published data soluble fractions of untreated aggregation reactions contained large numbers of predominantly spherical Aβ₄₂ oligomers with a diameter of 5-15 nm (Fig. 3e). In strong contrast, such structures were only very rarely detected in 04 treated samples. In the presence of 04, however, larger protofibrillar structures and amorphous Aβ₄₂ aggregates were predominantly observed, indicating that 04 binding to oligomers (Fig. 3c) converts such structures into larger aggregates. This result was confirmed when pellet fractions of 04 treated and untreated samples were analyzed by TEM (Fig. 3e). In the presence of 04 a relatively large number of long amyloid fibrils was detected in insoluble fractions, while such structures were not observed in the absence of the chemical compound. However, in pellet fractions of untreated aggregation reactions smaller protofibrillar aggregate species were detected by TEM (Fig. 3e).

The effect of 04 on early aggregate species was also visible when treated and untreated Aβ₄₂ preparations containing mainly small oligomers and protofibrils were analyzed after 24 h by SDS-PAGE and immunoblotting. We found that compound treatment stimulates the assembly of SDS-stable, higher molecular weight Aβ₄₂ aggregates in a concentration-dependent manner in cell-free assays (Fig. 3f), supporting the results observed by TEM (Fig. 3e).

Finally, we examined the effect of 04 on potentially toxic amyloid oligomers that are recognized by the conformation-specific antibody A11 (Kayed, 2003). Soluble Aβ₄₂ peptides were incubated in the presence and absence of 04 for 48 h at 37°C and the resulting aggregate species were analyzed by dot blot assays. As shown in Fig. 3g, A11-reactive oligomers were detected in the absence but not in the presence of the chemical compound (1:1 or 5:1 molar ratio), suggesting that 04-mediated acceleration of amyloidogenesis reduces the concentration of potentially toxic amyloid oligomers in spontaneous aggregation reactions (Kayed, 2003).

**2.2.5. 04 binds preferentially to hydrophobic regions in Aβ₄₂ peptides.** To define potential 04 binding regions in Aβ₄₂ peptides an array of 33 synthetic peptides representing the complete Aβ₄₂ sequence was prepared (Beutling, 2008) and probed for an interaction with the chemical compound. Each synthetic peptide was composed of 10 amino acids, which overlapped in sequence with the next peptide by 9 residues. Analysis of array binding data revealed that 04 most strongly interacts with Aβ₄₂ peptides, which contain non-polar, hydrophobic amino acids (e.g., peptide 12: VHHQKLVFFA (SEQ ID N0:1), aa 12-21 or peptide 24: VGSNKGAIIG (SEQ ID NO:2), aa 25-33, while peptide sequences with polar amino acids (e.g., peptide 1: DAEFRHDSGY (SEQ ID NO:3), aa 1-10) were not efficiently recognized (Fig. 6a). This suggests that 04 preferentially targets hydrophobic regions in Aβ₄₂ peptides, which are critical for efficient amyloid polymerization (Caputo, 1992; Hilbich, 1992).

We next investigated whether the position of hydrophobic amino acids in the Aβ₄₂ peptide sequence influences 04 binding. Two short peptides containing aromatic residues were synthesized (A: KLVFFAE, aa 16-22 of Aβ₄₂ and B: EFAVFLK, a scrambled version of A) and 04 binding was quantified colorimetrically. We observed that 04 readily interacts with peptide A but not with the control peptide B (Fig. 6b and c). In comparison, with the unspecific dye amido black both peptides were detected, indicating that similar amounts of material were spotted onto the filter membrane. This indicates that the interaction between 04 and the segments in Aβ₄₂ peptides is sequence specific and requires a certain order of hydrophobic amino acids.

**2.2.6. 04-generated amyloid fibrils have altered surface properties. We** next examined whether 04 treatment influences the morphology of mature amyloid fibrils. Aβ₄₂ peptides were incubated for 5 d at 37°C in the presence and absence of chemical compound and the resulting aggregates were analyzed with dot blot assays using the monoclonal antibodies 6E10 and 4G8, which specifically recognize amino acids 4-9 and 18-22 in Aβ₄₂ peptides, respectively (Kim, 1988). We observed that in comparison to untreated amyloid aggregates Aβ₄₂ aggregates generated in the presence of 04 were less efficiently recognized by the 4G8 antibody (Fig. 7a and c). This indicates that 04 binding to hydrophobic amino acids (Fig. 6a and b) alters the surface properties of amyloid aggregates and thereby reduces the binding of 4G8 to amino acids 18-22 in Aβ₄₂ peptides. Interestingly, such an effect was not observed with the monoclonal antibody 6E10 that recognizes the N-terminal amino acids 4-9 in Aβ₄₂ peptides, which do not interact with 04 in cell-free assays (Fig. 6a).

To confirm the observation that 04 alters surface properties of Aβ₄₂ aggregates, we also examined whether 04-generated Aβ₄₂ aggregates can be detected with the conformation specific antibody B10, which preferentially recognizes mature, β-sheet-rich amyloid fibrils and protofibrils, while monomers and low molecular oligomers are less efficiently detected (Habicht, 2007). As shown in Fig. 7b and d, addition of increasing concentrations of 04 to Aβ₄₂ aggregation reactions results in aggregate species with reduced B10 binding ability. Thus, our data indicate that 04 treatment in a concentration-dependent manner alters the surface properties of insoluble, β-sheet-rich Aβ₄₂ fibrils in cell free assays.

Analysis of amyloid fibrils by TEM and AFM also support the antibody binding results. While untreated Aβ₄₂ aggregates in agreement with previously published studies (Bieschke, 2005) are straight fibrils and show a plain, highly regular surface, such a fibrillar morphology was not detected with 04 treated samples (Fig. 7e and f). Addition of 04 to Aβ₄₂ aggregation reactions resulted in the assembly of mostly bent amyloid fibrils with a rough, irregular surface.

**2.2.7. Computational docking studies suggest that 04 binds to hydrophobic grooves on the surface of β-sheet-rich Aβ₄₂ protofibrils.** Our binding studies indicate that 04 directly associates with preformed β-sheet-rich amyloid fibrils in cell-free assays (Fig. 3c). We therefore employed a computational docking approach to assess how the compound might interact with fibrillar amyloid aggregates. We performed a series of automated blind 04 docking explorations utilizing a modeled 3D structure of an Aβ₄₂ protofibril (Lührs, 2005; Ahmed, 2010). To introduce some flexibility for the ligand the docking application Glide was applied (Friesner, 2006). Remarkably, in all adequate docking models the compound 04 was placed parallel to the long axis of the protofibril connecting 4-5 β-sheets (Fig. 8a). This suggests that 04 promotes amyloidogenesis because it stabilizes the intermolecular interactions of multiple Aβ₄₂ molecules.

We observed that 04 preferentially targets two hydrophobic binding grooves (aa 33-35 and aa 35-37) on the surface of Aβ₄₂ protofibrils. In addition, a reasonable, high scoring 04 binding site at amino acids 20-21 was predicted, suggesting that the compound has at least three favourable docking sites on the surface of amyloid fibrils (Fig. 8a and b). A detailed analysis revealed that the conjugated π-electron system of the planar oxazine ring in 04 most likely is critical for the association of the compound with hydrophobic grooves on the surface of Aβ₄₂ protofibrils. In case of the Aβ₄₂ binding site at aa 20-21 the oxazine ring of 04 might directly interact with the aromatic rings of phenylalanine 20 (F20, Fig. 8c), while at binding sites aa 33-35 and 35-37 an interaction between the oxacine ring and the glycines 33 and 37 is most likely. At all three favourable docking sites hydrogen bonds between hydroxyl groups in 04 and peptide carbonyl groups are also important for compound binding to amyloid protofibrils.

**2.2.8. 04-mediated acceleration of amyloidogenesis reduces Aβ₄₂ toxicity in cell-based assays and brain slices. To study whether O4-**mediated conversion of aggregation intermediates into stable amyloid fibrils influences Aβ₄₂ toxicity, SH-SY5Y neuroblastoma cells were incubated for 48 h with soluble Aβ₄₂ peptides and different concentrations of 04. Viability of cells was examined using a 3-(4,5-dimethylthiazol-2yl)-2,5-diphenyltetrazolium bromide (MTT) assay (Hansen, 1989). We found that incubation of cells with Aβ₄₂ peptides caused a pronounced inhibition of MTT reduction (∼50%), indicative of impaired cellular metabolic activity. This effect, however, was suppressed in a concentration-dependent manner when 04 was added to the culture medium (Fig. 9a).

To investigate whether 04 treatment influences Aβ₄₂ aggregate formation cell culture media were also analyzed by SDS-PAGE and silver staining (Fig. 9b). In-untreated samples, a relatively large amount of Aβ₄₂ monomers (-4 kDa) and small oligomers (-12 kDa) was detected, while in 04 treated samples the concentration of these Aβ₄₂ species was diminished. At the same time, an increase of higher molecular weight structures was observed in 04 treated preparations. These results were also confirmed when Aβ₄₂ treated primary cortical neurons were investigated. After addition of 04 (Fig. 10a and b) Aβ₄₂-mediated cellular toxicity was significantly reduced in neuronal cultures, which correlated with increased formation of larger protein aggregates (not shown).

Finally, we investigated whether 04 treatment influences Aβ₄₂-mediated impairment of hippocampal long-term potentiation (LTP) in rat brain slices (Wang, 2004; Walsh, 2002). A preparation of Aβ₄₂ peptides containing monomers and small oligomers was added to hippocampal brain slices and LTP was measured in the CA1 area. In agreement with previous studies (Wang, 2004), we found that small Aβ₄₂ oligomers block LTP in brain slices (Fig. 9c). This effect, however, was suppressed when 04 was added to samples, suggesting that compound-mediated acceleration of fibrillogenesis rescues the Aβ₄₂-induced blockage of synaptic transmission.

### 2.3. Discussion

Previous investigations have demonstrated that chemical agents can be used to perturb amyloid formation pathways (Heiser, 2000; Findeis, 2000). Most of these studies focused on small molecules or β-sheet breaker peptides that inhibit or slow down spontaneous amyloid polymerization (Hamaguchi et al. 2006; Soto et al., 1998). In this study, however, we followed a different strategy. We searched for small molecules that promote amyloid assembly aiming at compounds that target early events in the aggregation pathway of Aβ₄₂, a hydrophobic, short polypeptide, which causes neuronal dysfunction and degeneration in AD (Roher et al., 1993) The search for compounds that promote Aβ₄₂ aggregation was motivated by recent observations that small, diffusible aggregate species (oligomers and/or protofibrils) are more toxic for neuronal cells than mature fibrils or end-stage amyloid plaques (Lauren et al., 2009; Koffie et al., 2009). Accordingly, chemical compounds that directly target small, on-pathway aggregate species and promote their conversion into larger amyloid fibrils might reduce neurotoxicity in AD model systems.

Utilizing a membrane FRA that allows the quantification of large, insoluble amyloid aggregates (Wanker, 1999), we found that the natural dye orcein efficiently promotes Aβ₄₂ polymerization (Fig. 2a and b). Orcein is a compound mixture consisting of ∼14 related small molecules (Beecken et al., 2003) that is extracted from several species of lichen. Orcein was used as a food coloring since the Middle Ages, indicating that its components are of negligible toxicity for humans (Beecken et al., 2003). As orcein is a relatively complex mixture of related compounds that might have different biological activities, we next searched for purer chemicals with amyloid aggregation promoting properties. *In vitro* assays with orcein-related substances revealed that the synthetic compound O4 (2,8-bis-(2,4-dihydroxy-phenyl)-7-hydroxy-phenoxazin-3-one) is also a potent stimulator of Aβ₄₂ aggregation. In this study 04 was then utilized for detailed biochemical and biophysical investigations in order to elucidate its mechanism of action.

### 2.3.1. 04 targets small amyloid oligomers and converts them into larger fibrillar structures

Previous studies have demonstrated that Aβ₄₂ amyloidogenesis is a highly complex, multistep process, which involves the sequential formation of different types of amyloid species such as oligomers, protofibrils and mature fibrils (Jan et al., 2010). Aβ₄₂ monomers first assemble into densely packed spherical oligomers, which over time convert into elongated protofibrils and fibrils. While early Aβ₄₂ oligomers only contain partially formed β-sheet structures, such structures are a characteristic feature of protofibrils and fibrils (Kirschner et al., 1986). This indicates that the assembly of amyloid fibrils is accompanied by conformational changes in the aggregating proteins and that oligomers are both morphologically and structurally different from protofibrils and fibrils.

Recent studies have shown that relatively stable Aβ₄₂ oligomers are formed at low temperature that can convert into amyloid fibrils when they are incubated at higher temperature (Ahmed 2010). This supports the view that the small neurotoxic amyloid oligomers observed in brains of AD patients and transgenic animals (Walsh et al, 2002) are on-pathway aggregate species that might be converted into less toxic aggregates by compound treatment. Structural investigations with preformed Aβ₄₂ oligomers also revealed that they are composed of loosely aggregated peptides, which have a characteristic β-strand-turn-β-strand (β-turn-β) conformation (Ahmed, 2010). Strikingly, a very similar arrangement of (β-strands was also previously detected in Aβ₄₂ protofibrils, which have a characteristic β-sheet structure (Lührs et al., 2005). Thus, the fold of C-terminal hydrophobic Aβ₄₂ sequences in spherical oligomers and protofibrils is similar, although β-strands in amyloid oligomers do not have a stacked β-sheet structure but rather a piewedge shaped association of Aβ monomers (Ahmed 2010). It is not yet clear, whether these structures are directly *en route* to the deposition of amyloid plaques or whether they are in equilibrium with di- and tetrameric stacks of monomers that have previously been observed to be direct precursors of protofibril formation (Mastrangelo 2006).

Our data indicate that 04 directly binds to preformed Aβ₄₂ oligomers, while unstructured monomers are less efficiently recognized (Fig. 3c). Moreover, we observed that 04 binds to stretches of hydrophobic residues in the central and C-terminal region of Aβ₄₂ peptides (Fig. 6a). These regions were shown previously to be critical for the efficient formation of β-sheet-rich amyloid fibrils (Kim and Hecht, 2006). This data suggests that binding of 04 to hydrophobic regions on the surface of Aβ₄₂ oligomers stimulates their conversion into protofibrillar and fibrillar structures. Currently the mechanism by which this transition is promoted by 04 is unclear. We suggest that 04 binding to hydrophobic Aβ₄₂ segments alters hydrogen bonding of β-strands, which subsequently stabilizes the stacking of β-turn strands into a β-sheet secondary structure (Lührs et al., 2005). This conformation is a prerequisite of efficient amyloid polymerization, which is also supported by molecular dynamics simulations demonstrating that increasing the stability of β-conformers favours spontaneous amyloid fibrillogenesis (Pellarin and Caflish, 2006). However, the structural changes induced by 04 binding might also lead to the exposure of hydrophobic residues on the surface of oligomers where new Aβ₄₂ monomers can bind, promoting the assembly of protofibrillar structures.

Previous studies have demonstrated that the transition of oligomers into fibrils is temperature-dependent (Ahmed, 2010), suggesting that this process is entropy driven and that a release of bound water molecules form the hydrophobic surface of spherical particles is critical for amyloid polymerization. Interestingly, our investigations have shown that 04 at low temperatures can promote the conversion of oligomers into fibrillar structures (Fig. 3e). This indicates that in the presence of 04 higher temperatures are not required to convert Aβ₄₂ oligomers into amyloid fibrils. We suggest that the binding of the aromatic compound 04 helps to remove water molecules from the surface of oligomers and thereby increases their hydrophobicity, which subsequently leads to a faster assembly of larger aggregates. However, we also suggest that π-stacking interactions with aromatic amino acids in the Aβ₄₂ peptides could be responsible for the association of 04 with oligomers (Gazit, 2002). Previous studies have demonstrated that two phenylalanine residues (F19 and F20) in Aβ₄₂ are critical for efficient amyloid polymerization (Kim and Hecht, 2006). This is also supported by observations that short peptides with phenylalanines are potent inhibitors of amyloid polymerization *in vitro* and *in vivo* (Soto, 1998). 04 binding to peptide arrays indicates that these aromatic amino acids indeed might be critical for the 04-mediated conversion of oligomers into protofibrils. However, our peptide array data also show that more C-terminal hydrophobic amino acids (e.g. G33, G37) could be responsible for compound binding. Detailed structural studies with preformed Aβ₄₂ oligomers and 04 are necessary to elucidate the exact compound binding site and the mechanism by which oligomers are potentially converted into protofibrils.

### 2.3.2. 04 directly targets protofibrils and promotes their assembly into mature fibrils

Our data indicate that 04 also binds to protofibrils and mature amyloid fibrils with a characteristic β-sheet structure (Fig. 3c). This is supported also by computational docking studies that suggest 04 to bind to distinct hydrophobic grooves on the surface of protofibrils (aa 20-21, 33-35, 35-37) that run parallel to the long fibril axis (Fig. 8a and b). Previously, a model for a potential high-resolution 3D structure of Aβ₄₂ protofibrils has been put forward (Lührs et al., 2005) which has more recently been refined (Paravastu, 2008; Tycko, 2006; Ahmed, 2010). It indicates that amyloid fibrils are composed of multiple β-turn-β units polymerizing in a parallel and in-register orientation. While the central and C-terminal hydrophobic residues of Aβ₄₂ (aa 12-42) are structured and highly important for spontaneous amyloid assembly, the polar N-terminal residues (aa 1-11) are unstructured and not required for fibrillization. Our results that 04 binds to the structured, hydrophobic amino acids but not to polar residues at the N-terminus of Aβ₄₂ (Fig. 6 and 8) support previous studies that hydrophobic stretches of central and C-terminal amino acids are critical for efficient Aβ₄₂ polymerization (Kim and Hecht, 2006).

Strikingly, our computational docking studies indicate that 04 requires 4-5 repeated β-turn-β units for its association with Aβ₄₂ protofibrils (Fig. 8). This suggests that compound binding stabilizes seeding-competent protofibrils with at least 4 assembled Aβ₄₂ molecules in complex, heterogenous aggregation reactions. Thus, 04 could stimulate fibrillogenesis because it stabilizes structures that lie directly en route to fibril formation and thereby alters the equilibrium between monomers, oligomers and protofibrils. Our findings that 04 treated amyloid fibrils are more stable than untreated fibrils support this hypothesis (Fig. 3a and b). Moreover, we observed that O4-generated amyloid fibrils are even better seeds than untreated aggregates (Fig. 2g). Thus, 04 binding to protofibrils and/or fibrils could reduce the lag phase of amyloid polymerization (Jarrett et al., 1993) because aggregation-competent seeds are formed faster and are more stable. Moreover, it could promote fibril growth because 04-generated seeds can bind Aβ₄₂ monomers more efficiently than untreated seeds. A model depicting the binding of 04 to protofibrils and fibrils and its potential effects on amyloid polymerization is shown in Fig. 11.

Our docking models also suggest that 04 binding to Aβ₄₂ protofibrils might stabilize the lateral association of such structures into mature amyloid fibrils (Fig. 12). This supports our experimental results that 04 treatment leads to the formation of highly, stable amyloid fibrils with tightly bound compound molecules, which cannot be removed from aggregates by treatment with detergents (data not shown).

Previous studies indicate that the dye ThT also associates with hydrophobic grooves on the surface of Aβ₄₂ amyloid aggregates that run parallel to the fibril axis (Biancalana and Koide, 2010). Interestingly, for both ThT and 04 potential binding sites at glycine residues 33 and 37 were predicted by computational docking studies, suggesting that these compounds target similar locations on the surface of amyloid aggregates. However, more detailed biochemical and structural studies with amyloid binding compounds such as ThT, 04 and Congo red are required in order to elucidate whether they target similar sites on the surface of protofibrils and mature amyloid fibrils.

### 2.3.3. Implications for neurodegenerative diseases

Our studies indicate that the compound 04 accelerates Aβ₄₂ fibril formation in cell-free and cell-based disease model systems and thereby decreases the concentration of transient, soluble Aβ₄₂ oligomers that are toxic for mammalian cells. Thus, our biochemical and cell-biological investigations support the oligomer-toxicty hypothesis, suggesting that small aggregates appearing early in the amyloid formation cascade rather than large fibrils are the main toxic species in protein misfolding diseases (Walsh et al., 2002). Studies with the protein Pmel17, which forms a functional amyloid structure that scaffolds melanin polymerization, demonstrated that rapid fibril assembly is a hallmark of functional amyloids that reduces the toxicity of amyloid formation (Fowler et al., 2006). However, *in vivo* studies with AD transgenic mouse and fly models will be necessary to investigate whether compounds that promote fibrillogenesis such as 04 are indeed useful for therapy development. Previous biochemical investigations indicate that the compound methylene blue, which influences tau aggregation (Wischik et al., 1996), similar to 04also promotes Aβ₄₂ fibrillogenesis and thereby reduces the amount of A11-reactive, potentially toxic amyloid oligomers (Necula et al., 2007). Strikingly, methylene blue was successful in phase II clinical AD trials, suggesting that compounds that directly target amyloid formation pathways could be of therapeutic benefit for patients. On the basis of the mechanistic studies presented here, we conclude that 04 and perhaps other related compounds have considerable potential as drugs for the treatment of amlyloid diseases such as AD.

### References

Ahmed M, Davis J, Aucoin D, Sato T, Ahuja S, Aimoto S, Elliott JI, Van Nostrand WE, Smith SO. (2010) "Structural conversion of neurotoxic amyloid-beta(1-42) oligomers to fibrils." Nat Struct Mol Biol 17(5):561-7.
Beecken, H., E. M. Gottschalk, et al. (2003). "Orcein and litmus." Biotech Histochem 78(6): 289-302.
Beutling, U., K. Städing, et al. (2008). "Large-Scale Analysis of Protein-Protein Interactions Using Cellulose-Bound Peptide Arrays", Adv Biochem Eng Biotechnol 110:115-52
Biancalana, M. and S. Koide (2010) "Molecular mechanism of Thioflavin-T binding to amyloid fibrils." Biochim Biophys Acta 1804(7): 1405-12.
Bieschke, J., Q. Zhang, et al. (2005). "Oxidative metabolites accelerate Alzheimer's amyloidogenesis by a two-step mechanism, eliminating the requirement for nucleation." Biochemistry 44(13): 4977-83.
Bieschke, J., J. Russ, et al. (2010). "EGCG remodels mature alpha-synuclein and amyloid-beta fibrils and reduces cellular toxicity." Proc Natl Acad Sci U S A 107(17): 7710-5.
Bitan, G., M. D. Kirkitadze, et al. (2003). "Amyloid beta -protein (Abeta) assembly: Abeta 40 and Abeta 42 oligomerize through distinct pathways." Proc Natl Acad Sci U S A 100(1): 330-5.
Bucciantini, M., E. Giannoni, et al. (2002). "Inherent toxicity of aggregates implies a common mechanism for protein misfolding diseases." Nature 416(6880): 507-11.
Caputo, C. B., P. E. Fraser, et al. (1992). "Amyloid-like properties of a synthetic peptide corresponding to the carboxy terminus of beta-amlyloid protein precursor." Arch Biochem Biophys 292(1): 199-205.
Cohen, F. E. and J. W. Kelly (2003). "Therapeutic approaches to protein-misfolding diseases." Nature 426(6968): 905-9.
Conway, K. A., J. C. Rochet, et al. (2001). "Kinetic stabilization of the alpha-synuclein protofibril by a dopamine-alpha-synuclein adduct." Science 294(5545): 1346-9.
Dobson, C. M. (2003). "Protein folding and misfolding." Nature 426(6968): 884-90.
Ehrnhoefer, D. E., J. Bieschke, et al. (2008). "EGCG redirects amyloidogenic polypeptides into unstructured, off-pathway oligomers." Nat Struct Mol Biol 15(6): 558-66.
Ehrnhoefer, D. E., M. Duennwald, et al. (2006). "Green tea (-)-epigallocatechin-gallate modulates early events in huntingtin misfolding and reduces toxicity in Huntington's disease models." Hum Mol Genet 15(18): 2743-51.
Findeis, M. A. (2000). "Approaches to discovery and characterization of inhibitors of amyloid beta-peptide polymerization." Biochim Biophys Acta 1502(1): 76-84.
Fowler, D. M., A. V. Koulov, et al. (2006). "Functional amyloid formation within mammalian tissue." PLoS Biol 4(1): e6.
Friesner, R. A., R. B. Murphy, et al. (2006). "Extra precision glide: docking and scoring incorporating a model of hydrophobic enclosure for protein-ligand complexes." J Med Chem 49(21): 6177-96.
Gazit, E. (2002). "A possible role for pi-stacking in the self-assembly of amyloid fibrils." FASEB J 16(1): 77-83.
Habicht, G., C. Haupt, et al. (2007). "Directed selection of a conformational antibody domain that prevents mature amyloid fibril formation by stabilizing Abeta protofibrils." Proc Natl Acad Sci U S A 104(49): 19232-7.
Hamaguchi, T., K. Ono, et al. (2006). "Anti-amyloidogenic therapies: strategies for prevention and treatment of Alzheimer's disease." Cell Mol Life Sci 63(13): 1538-52.
Hansen, M. B., S. E. Nielsen, et al. (1989). "Re-examination and further development of a precise and rapid dye method for measuring cell growth/cell kill." J Immunol Methods 119(2): 203-10.
Harper, J. D. and P. T. Lansbury, Jr. (1997). "Models of amyloid seeding in Alzheimer's disease and scrapie: mechanistic truths and physiological consequences of the time-dependent solubility of amyloid proteins." Annu Rev Biochem 66: 385-407.
Harper, J. D., S. S. Wong, et al. (1999). "Assembly of A beta amyloid protofibrils: an in vitro model for a possible early event in Alzheimer's disease." Biochemistry 38(28): 8972-80.
Heiser, V., S. Engemann, et al. (2002). "Identification of benzothiazoles as potential polyglutamine aggregation inhibitors of Huntington's disease by using an automated filter retardation assay." Proc Natl Acad Sci U S A 99 Suppl 4: 16400-6.
Herbst, M. and E. E. Wanker (2006). "Therapeutic approaches to polyglutamine diseases: combating protein misfolding and aggregation." Curr Pharm Des 12(20): 2543-55.
Hilbich, C., B. Kisters-Woike, et al. (1992). "Substitutions of hydrophobic amino acids reduce the amyloidogenicity of Alzheimer's disease beta A4 peptides." J Mol Biol 228(2): 460-73.
Jan, A., D. M. Hartley, et al. (2010)"Preparation and characterization of toxic Abeta aggregates for structural and functional studies in Alzheimer's disease research." Nat Protoc 5(6): 1186-209.
Jarrett, J. T. and P. T. Lansbury, Jr. (1993). "Seeding "one-dimensional crystallization" of amyloid: a pathogenic mechanism in Alzheimer's disease and scrapie?" Cell 73(6): 1055-8.
Kayed, R., E. Head, et al. (2003). "Common structure of soluble amyloid oligomers implies common mechanism of pathogenesis." Science 300(5618): 486-9.
Kim, W. and M. H. Hecht (2006). "Generic hydrophobic residues are sufficient to promote aggregation of the Alzheimer's Abeta42 peptide." Proc Natl Acad Sci U S A 103(43): 15824-9.
Kim, K. S. (1988). "Production and characterization of monoclonal antibodies reactive to synthetic cerebrovascular amyloid peptide." Neuroscience Research Communications 2: 121-130.
Kirschner, D. A., C. Abraham, et al. (1986). "X-ray diffraction from intraneuronal paired helical filaments and extraneuronal amyloid fibers in Alzheimer disease indicates cross-beta conformation." Proc Natl Acad Sci U S A 83(2): 503-7.
Koffie, R. M., M. Meyer-Luehmann, et al. (2009). "Oligomeric amyloid beta associates with postsynaptic densities and correlates with excitatory synapse loss near senile plaques." Proc Natl Acad Sci U S A 106(10): 4012-7.
Lambert, M. P., A. K. Barlow, et al. (1998). "Diffusible, nonfibrillar ligands derived from Abeta1-42 are potent central nervous system neurotoxins." Proc Natl Acad Sci U S A 95(11): 6448-53.
LeVine, H. (2002). "The challenge of inhibiting Abeta polymerization." Curr Med Chem 9(11): 1121-33.
Lauren, J., D. A. Gimbel, et al. (2009). "Cellular prion protein mediates impairment of synaptic plasticity by amyloid-beta oligomers." Nature 457(7233): 1128-32.
Lührs, T., Ritter, C., Adrian, M., Riek-Loher, D., Bohrmann, B., Dobeli, H., Schubert, D. & Riek, R. (2005) "3D structure odf Alzheimer's amyloid-beta(1-42) fibrils." Proc Natl Acad Sci U S A 102(48): 17342-7.
Ma, Q. L., G. P. Lim, et al. (2006). "Antibodies against beta-amyloid reduce Abeta oligomers, glycogen synthase kinase-3beta activation and tau phosphorylation in vivo and in vitro." J Neurosci Res 83(3): 374-84.
Mastrangelo, I. A., M. Ahmed, et al. (2006). "High-resolution atomic force microscopy of soluble Abeta42 oligomers." J Mol Biol 358(1): 106-19.
Necula, M., R. Kayed, et al. (2007). "Small molecule inhibitors of aggregation indicate that amyloid beta oligomerization and fibrillization pathways are independent and distinct." J Biol Chem 282(14): 10311-24.
Paravastu, A. K., R. D. Leapman, R.D., Yau, W.M. & Tycko, R. (2008), "Molecular structural basis for polymorphism in Alzheimer's beta-amyloid fibrils." Proc Natl Acad Sci U S A 105(47): 18349-54.
Pellarin, R. and A. Caflisch (2006). "Interpreting the aggregation kinetics of amyloid peptides." J Mol Biol 360(4): 882-92.
Roher, A. E., J. D. Lowenson, et al. (1993). "beta-Amyloid-(1-42) is a major component of cerebrovascular amyloid deposits: implications for the pathology of Alzheimer disease." Proc Natl Acad Sci U S A 90(22): 10836-40.
Soto, C. (1999). "Plaque busters: strategies to inhibit amyloid formation in Alzheimer's disease." Mol Med Today 5(8): 343-50.
Soto, C., E. M. Sigurdsson, et al. (1998). "Beta-sheet breaker peptides inhibit fibrillogenesis in a rat brain model of amyloidosis: implications for Alzheimer's therapy." Nat Med 4(7): 822-6.
Tycko R. (2006). "Characterization of amyloid structures at the molecular level by solid state nuclear magnetic resonance spectroscopy." Methods Enzymol 413:103-22.
Walsh, D. M., I. Klyubin, et al. (2002). "Naturally secreted oligomers of amyloid beta protein potently inhibit hippocampal long-term potentiation in vivo." Nature 416(6880): 535-9.
Walsh, D. M., A. Lomakin, et al. (1997). "Amyloid beta-protein fibrillogenesis. Detection of a protofibrillar intermediate." J Biol Chem 272(35): 22364-72.
Walsh, D. M., A. M. Minogue, et al. (2007). "The APP family of proteins: similarities and differences." Biochem Soc Trans 35(Pt 2): 416-20.
Walsh, D. M. and D. J. Selkoe (2007). "A beta oligomers - a decade of discovery." J Neurochem 101(5): 1172-84.
Walsh, D.M. D. M. Hartley, et al. (1999). "Amyloid beta-protein fibrillogenesis. Structure and biological activity of protofibrillar intermediates." J Biol Chem 274(36): 25945-52.
Wang, Q., D. M. Walsh, et al. (2004). "Block of long-term potentiation by naturally secreted and synthetic amyloid beta-peptide in hippocampal slices is mediated via activation of the kinases c-Jun N-terminal kinase, cyclin-dependent kinase 5, and p38 mitogen-activated protein kinase as well as metabotropic glutamate receptor type 5." J Neurosci 24(13): 3370-8.
Wanker, E. E., E. Scherzinger, et al. (1999). "Membrane filter assay for detection of amyloid-like polyglutamine-containing protein aggregates." Methods Enzymol 309: 375-86.
Williams, A. D., M. Sega, et al. (2005). "Structural properties of Abeta protofibrils stabilized by a small molecule." Proc Natl Acad Sci U S A 102(20): 7115-20.
Wischik, C. M., P. C. Edwards, et al. (1996). "Selective inhibition of Alzheimer disease-like tau aggregation by phenothiazines." Proc Natl Acad Sci U S A 93(20): 11213-8.
Yang, D. S., C. M. Yip, et al. (1999). "Manipulating the amyloid-beta aggregation pathway with chemical chaperones." J Biol Chem 274(46): 32970-4.
Yang, W., J. R. Dunlap, et al. (2002). "Aggregated polyglutamine peptides delivered to nuclei are toxic to mammalian cells." Hum Mol Genet 11 (23): 2905-17.

## Claims

1. A compound of formula (I), 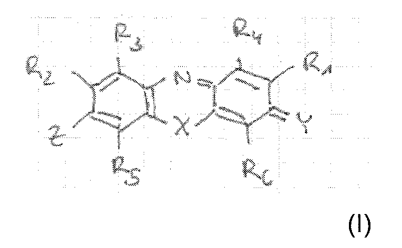 wherein
XisOorS,
Y is O or NR⁷,
Z is OR⁷ or N(R⁷)₂,
at least one of R¹ and R² is C₃₋₈ cyclic group substituted with at least one OH group and optionally other substituents,
R¹, R², R³, R⁴, R⁵ and R⁶ are in each case independently selected from H, halogen or C₁₋₃ alkyl or C₁₋₃ alkoxy optionally substituted with halogen,
R⁷ is in each case independently selected from H and C₁₋₃ alkyl optionally substituted with halogen, or
a pharmaceutically acceptable salt thereof for use in medicine.

2. The compound of claim 1, wherein X is O.

3. The compound of claim 1 or 2, wherein
(i) Y is O and Z is OR⁷,
(ii) Y is O and Z is N(R⁷)₂,
(iii) Y is NR⁷ and Z is OR⁷, or
(iv) Y is NR⁷ and Z is N(R⁷)₂.

4. The compound of any one of claims 1-3, wherein
(i) only R¹ is a cyclic group,
(ii) only R² is a cyclic group or
(iii) R¹ and R² are both cyclic groups.

5. The compound of any one of claims 1-4, wherein at least one of R¹ and R² is phenyl substituted with at least one, e.g. one or two OH groups and optionally other substituents.

6. The compound of any one of claims 1-5, wherein R⁷ is H.

7. The compound of any one of claims 1-6, which is selected from
α -amino orcein,
α -hydroxy orcein,
β -amino orcein,
β -amino orceimine,
γ -amino orcein,
γ -amino orceimine,
2,8-bis (2,4-dihydroxy-phenyl)-7-hydroxy-phenazin-3-one or
a pharmaceutically acceptable salt thereof.

8. The compound of any one of claims 1-7, which is 2,8-bis (2,4-di-hydroxy-phenyl)-7-hydroxy-phenazin-3-one or a pharmaceutically acceptable salt thereof.

9. The compound of any one of claims 1-8 for use in the acceleration of amyloid-β fibril formation.

10. The compound of any one of claims 1-9 for use in the inhibition of amyloid-β oligomer formation.

11. The compound of any one of claims 1-10 for use in the treatment of amyloid diseases.

12. The compound of any one of claims 1-11 for use in the treatment of Alzheimer's disease.

13. The compound of any one of claims 1-8 for use in diagnosis of amyloid diseases.

14. The compound of any one of claims 1-8 for use in diagnosis of Alzheimer's disease.
